# EUROPEAN PATENT APPLICATION

(11) **EP 3 572 430 A2**
(43) Date of publication of application: **27.11.2019**
(21) Application number: 19167467.0
(22) Date of filing: 04.03.2015
(51) Int. Cl.: C07K 16/28, A61K 31/404, A61K 31/44, A61K 39/395, A61P 35/00

(54) **TREATMENT OF RENAL CANCER USING A COMBINATION OF AN ANTI-PD-1 ANTIBODY AND ANOTHER ANTI-CANCER AGENT**

(30) Priority: 05.03.2014 US 201461948428 P; 09.04.2014 US 201461977318 P; 13.05.2014 US 201461992729 P; 30.05.2014 US 201462005600 P
(62) Divisional of application: 15710681.6
(71) Applicant: Bristol-Myers Squibb Company, Princeton, NJ 08543 (US)
(72) Inventor: Jure-Kunkel, Maria, Princeton, New Jersey 08543 (US); Gagnier, Paul, Meriden, Connecticut 06460 (US); Feltquate, David, Princeton, New Jersey 08543 (US)
(74) Representative: Reitstötter Kinzebach

(57) **Abstract**

This disclosure provides a method for treating a subject afflicted with a renal cancer, which method comprises administering to the subject therapeutically effective amounts of: (a) an anti-cancer agent which is an antibody or an antigen-binding portion thereof that specifically binds to a Programmed Death-1 (PD-1) receptor and inhibits PD-1 activity; and (b) another anti-cancer agent. The other anti-cancer agent may be an anti-angiogenic tyrosine kinase inhibitor or an anti-Cytotoxic T-Lymphocyte Antigen-4 (CTLA-4) antibody. The disclosure also provides a kit for treating a subject afflicted with a renal cancer, the kit comprising a dosage of an anti-PD-1 antibody, a dosage of another anti-cancer agent which is an anti-angiogenic tyrosine kinase inhibitor or an anti-CTLA-4 antibody, and instructions for using the anti-PD-1 antibody and the other anti-cancer agent in any of the disclosed methods for treating a renal cancer.

## Description

Throughout this application, various publications are referenced in parentheses by author name and date, or by Patent No. or Patent Publication No. Full citations for these publications may be found at the end of the specification immediately preceding the claims. The disclosures of these publications are hereby incorporated in their entireties by reference into this application in order to more fully describe the state of the art as known to those skilled therein as of the date of the invention described and claimed herein. However, the citation of a reference herein should not be construed as an acknowledgement that such reference is prior art to the present invention.

### FIELD OF THE INVENTION

This invention relates to methods for treating renal cancer in a subject comprising administering to the subject a combination of an anti-cancer agent which is an anti-Programmed Death-1 (PD-1) antibody and another anti-cancer agent. In certain embodiments, the other anti-cancer agent is an anti-Cytotoxic T-Lymphocyte Antigen-4 (CTLA-4) antibody, an anti-angiogenic tyrosine kinase inhibitor, or a combination thereof.

### BACKGROUND OF THE INVENTION

Human cancers harbor numerous genetic and epigenetic alterations, generating neoantigens potentially recognizable by the immune system (Sjoblom *et al.,* 2006). The adaptive immune system, comprised of T and B lymphocytes, has powerful anti-cancer potential, with a broad capacity and exquisite specificity to respond to diverse tumor antigens. Further, the immune system demonstrates considerable plasticity and a memory component. The successful harnessing of all these attributes of the adaptive immune system would make immunotherapy unique among all cancer treatment modalities.

Until recently, cancer immunotherapy had focused substantial effort on approaches that enhance anti-tumor immune responses by adoptive-transfer of activated effector cells, immunization against relevant antigens, or providing non-specific immune-stimulatory agents such as cytokines. In the past decade, however, intensive efforts to develop specific immune checkpoint pathway inhibitors have begun to provide new immunotherapeutic approaches for treating cancer, including the development of an antibody (Ab), ipilimumab (YERVOY®), that binds to and inhibits Cytotoxic T-Lymphocyte Antigen-4 (CTLA-4) for the treatment of patients with advanced melanoma (Hodi *et al.,* 2010) and the development of Abs such as nivolumab and pembrolizumab (formerly lambrolizumab; USAN Council Statement, 2013) that bind specifically to the Programmed Death-1 (PD-1) receptor and block the inhibitory PD-1/PD-1 ligand pathway (Topalian *et al.,* 2012a, b; Topalian *et al.,* 2014; Hamid *et al.,* 2013; Hamid and Carvajal, 2013; McDermott and Atkins, 2013).

PD-1 is a key immune checkpoint receptor expressed by activated T and B cells and mediates immunosuppression. PD-1 is a member of the CD28 family of receptors, which includes CD28, CTLA-4, ICOS, PD-1, and BTLA. Two cell surface glycoprotein ligands for PD-1 have been identified, Programmed Death Ligand-1 (PD-L1) and Programmed Death Ligand-2 (PD-L2), that are expressed on antigen-presenting cells as well as many human cancers and have been shown to downregulate T cell activation and cytokine secretion upon binding to PD-1. Inhibition of the PD-1/PD-L1 interaction mediates potent antitumor activity in preclinical models (U.S. Patent Nos. 8,008,449 and 7,943,743), and the use of Ab inhibitors of the PD-1/PD-L1 interaction for treating cancer has entered clinical trials (Brahmer *et al.,* 2010; Topalian *et al.,* 2012a; Topalian *et al.,* 2014; Hamid *et al.,* 2013; Brahmer *et al.,* 2012; Flies *et al.,* 2011; Pardoll, 2012; Hamid and Carvajal, 2013).

Nivolumab (formerly designated 5C4, BMS-936558, MDX-1106, or ONO-4538) is a fully human IgG4 (S228P) PD-1 immune checkpoint inhibitor Ab that selectively prevents interaction with PD-1 ligands (PD-L1 and PD-L2), thereby blocking the down-regulation of antitumor T-cell functions (U.S. Patent No. 8,008,449; Wang et al., 2014). Nivolumab has shown activity in a variety of advanced solid tumors including renal cell carcinoma (RCC; renal adenocarcinoma, or hypernephroma), melanoma, and non-small cell lung cancer (Topalian *et al.,* 2012a; Topalian *et al.,* 2014; Drake *et al.,* 2013; WO 2013/173223).

Ipilimumab (YERVOY®) is a fully human, IgG1 monoclonal Ab that blocks the binding of CTLA-4 to its B7 ligands, thereby stimulating T cell activation and improving overall survival (OS) in patients with advanced melanoma (Hodi *et al.,* 2010). Concurrent therapy with nivolumab and ipilimumab in a Phase 1 clinical trial produced rapid and deep tumor regression in a substantial proportion of patients with advanced melanoma, and was significantly more effective than either Ab alone (Wolchok et al., 2013; WO 2013/173223). However, it was hitherto not known whether this combination of immunoregulatory Abs would be similarly effective in other tumor types.

Renal cancer (also known as kidney cancer) is a cancer that originates in the kidneys. The most common type of kidney cancer is renal cell carcinoma (RCC). RCC can often be cured by surgical resection if it is diagnosed and treated when still localized to the kidney and to the immediately surrounding tissue (Stage I), and radical resection is the accepted, often curative, therapy for Stage II as well as Stage III RCC. In contrast, when distant metastases are present (Stage IV), disease-free survival is poor. Moreover, the prognosis for any treated RCC patient with progressing, recurring, or relapsing disease is also poor, regardless of cell type or stage. Approximately 25%-30% of RCC patients have metastatic disease at diagnosis, and median survival for metastatic RCC is only about 24 months (Gupta *et al.,* 2008; NCCN GUIDELINES®, Version 3.2014 - Kidney Cancer; Heng *et al.,* 2009). Responses to cytotoxic chemotherapy generally have not exceeded 10% for any regimen that has been studied in adequate numbers of patients. However, a growing understanding of the biology of RCC has led to the development and U.S. Food and Drug Administration (FDA) approval of seven new agents targeting specific growth pathways. Two of the approved targeted therapies, temsirolimus and everolimus, block the mammalian target of rapamycin (mTOR), a serine/threonine protein kinase that regulates cell growth, division, and survival. Several agents that target vascular endothelial growth factor (VEGF)-mediated pro-angiogenesis pathways have been developed, including five oral tyrosine kinase inhibitors (TKIs), pazopanib, sorafenib, sunitinib, axitinib and tivozanib, and an anti-VEGF monoclonal antibody (mAb), bevacizumab. These drugs show clinical efficacy and improved progression-free survival (PFS), but durable response are rare. VEGF TKIs have been shown to suppress immune-inhibitory regulatory T cells (Tregs) and myeloid-derived suppressor cells (MDSCs), making the immune environment more conducive for T cell-mediated antitumor activity. Moreover, targeting the VEGF axis may attenuate RCC-induced immunosuppression, allowing the tumor to become more responsive to immune modulation when used in combination, and thereby resulting in greater and more durable therapeutic benefit.

As described herein, the activity of nivolumab in combination with two anti-angiogenic (anti-VEGF) TKIs, sunitinib (SUTENT®) or sorafenib (NEXAVAR®), has been investigated in a preclinical RCC murine model. Clinical data on the combination of nivolumab with sunitinib, pazopanib (VOTRIENT®), or ipilimumab are also provided herein.

### SUMMARY OF THE INVENTION

The present disclosure provides a method for treating a subject afflicted with a renal cancer comprising administering to the subject a combination of therapeutically effective amounts of: (a) an Ab or an antigen-binding portion thereof that specifically binds to and inhibits PD-1; and (b) another anti-cancer agent. In certain embodiments, the other anti-cancer agent is either (i) an Ab or an antigen-binding portion thereof that specifically binds to and inhibits CTLA-4, (ii) an anti-angiogenic TKI, or (iii) a combination thereof. In some embodiments, the renal cancer is RCC. In certain embodiments of any of the therapeutic methods disclosed herein, the anti-PD-1 Ab is nivolumab. In other embodiments, the anti-PD-1 Ab is pembrolizumab. In certain other embodiments of any of the therapeutic methods disclosed herein, the anti-CTLA-4 Ab is ipilimumab. In other embodiments, the anti-CTLA-4 Ab is tremelimumab. In yet other embodiments, the TKI is sunitinib, pazopanib, sorafenib, axitinib or tivozanib.

In certain embodiments, the subject has been pre-treated for the renal cancer. In other embodiments, the renal cancer is an advanced, metastatic and/or refractory cancer. In some embodiments, the administration of the combination of the Ab or antigen-binding portion thereof and the TKI induces a durable clinical response in the subject.

In certain embodiments, the administration of the combination of the anti-PD-1 Ab or antigen-binding portion thereof and the other anti-cancer agent results in an increase in T cell infiltration of the renal cancer tissue or tumor relative to an untreated subject or to a subject treated with a monotherapy of the anti-PD-1 Ab or antigen-binding portion thereof or the other anti-cancer agent. In other embodiments, the increased T cell infiltration is characterized by an increased infiltration of CD4⁺ T cells, CD8⁺ T cells, or both into the renal cancer tissue or tumor. In still other embodiments, the administration of the combination of the anti-PD-1 Ab or antigen-binding portion thereof and another anti-cancer agent results in increased T cell proliferation. In another embodiment, the administration of the combination of the anti-PD-1 Ab or antigen-binding portion thereof and the other anti-cancer agent results in a decrease in T-regulatory cells relative to an untreated subject or a subject treated with a monotherapy of an anti-PD-1 Ab or antigen-binding portion thereof or the other anti-cancer agent.

In certain embodiments, administration of the combination of the anti-PD-1 Ab or antigen-binding portion thereof and another anti-cancer agent decreases the number of monocytic myeloid-derived suppressor cells. In some embodiments, administration of the combination of the anti-PD-1 Ab or antigen-binding portion thereof and another anti-cancer agent increases the number of granulocytic myeloid cells, relative to an untreated subject or a subject treated with a monotherapy of an anti-PD-1 Ab or antigen-binding portion thereof or the other anti-cancer agent. In some embodiments, the monocytic myeloid-derived suppressor cells are characterized by expression of CD11b⁺/Ly6C^{hi}/Ly6G⁻ or by expression of CD11b⁺/Ly6C^{low}Ly6G⁻. In other embodiment, the granulocytic myeloid cells are characterized by expression of CD11b⁺/Ly6C⁻/Ly6G⁺.

In certain embodiments of the methods comprising use of an anti-PD-1 Ab or antigen-binding portion thereof in combination with a TKI, the therapeutically effective dosage of the anti-PD-1 Ab or antigen-binding portion thereof ranges from about 0.1 to about 10.0 mg/kg body weight administered by intravenous infusion once about every 2-4 weeks. In certain embodiments, the anti-PD-1 Ab or antigen-binding portion thereof is administered at a dose of about 2 mg/kg or about 5 mg/kg once every three weeks.

For the combination of an anti-PD-1 Ab and an anti-CTLA-4 Ab, the method comprises in certain embodiments (a) an induction phase comprising 4 doses of the anti-PD-1 and anti-CTLA-4 Abs administered at 3-week intervals at the following dosages: (i) about 1 mg/kg anti-PD-1 Ab and about 3 mg/kg of anti-CTLA-4 Ab; (ii) about 3 mg/kg anti-PD-1 Ab and about 3 mg/kg of anti-CTLA-4 Ab; or (iii) about 3 mg/kg anti-PD-1 Ab and about 1 mg/kg of anti-CTLA-4 Ab; and (b) a maintenance phase comprising repeated administration of the anti-PD-1 Ab at a dose of about 3 mg/kg every 2 weeks.

The disclosure also provides a kit for treating a subject afflicted with a renal cancer, the kit comprising: (a) a dosage of an Ab or an antigen-binding portion thereof that specifically binds to and inhibits PD-1; (b) one of a dosage of (i) an Ab or an antigen-binding portion thereof that specifically binds to and inhibits CTLA-4 or (ii) an anti-angiogenic TKI, for example, sunitinib or pazopanib; and (c) instructions for using the anti-PD-1 Ab and the anti-CTLA-4 Ab or TKI for treating the subject.

Other features and advantages of the instant invention will be apparent from the following detailed description and examples which should not be construed as limiting. The contents of all cited references, including scientific articles, newspaper reports, GenBank entries, patents and patent applications cited throughout this application are expressly incorporated herein by reference.

### BRIEF DESCRIPTION OF THE FIGURES

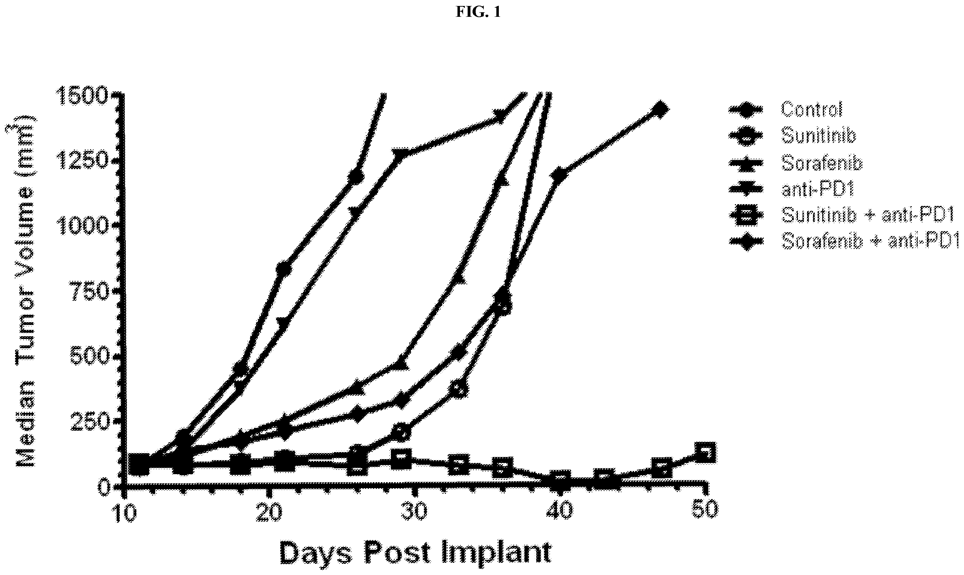

Figure 1 shows the effects of treatment with the following therapeutic agents on mean tumor volume growth: control (filled circle), sunitinib (open circle), sorafenib (filled triangle), anti-PD-1 (filled inverted triangle), sunitinib + anti-PD-1 (open square), and sorafenib + anti-PD-1 (filled diamond).

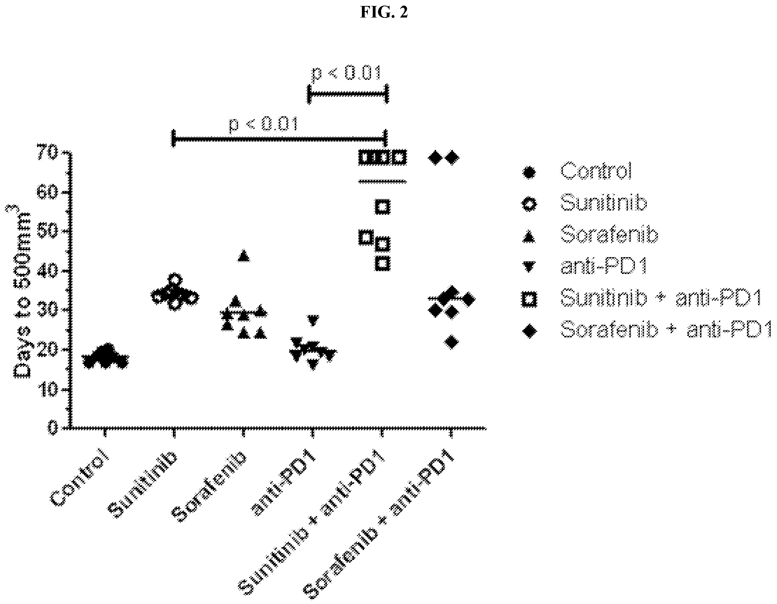

Figure 2 shows the time taken for tumors treated with control (filled circle), sunitinib (open circle), sorafenib (filled triangle), anti-PD-1 (filled inverted triangle), sunitinib + anti-PD-1 (open square), and sorafenib + anti-PD-1 (filled diamond), to reach a volume of 500 mm³.

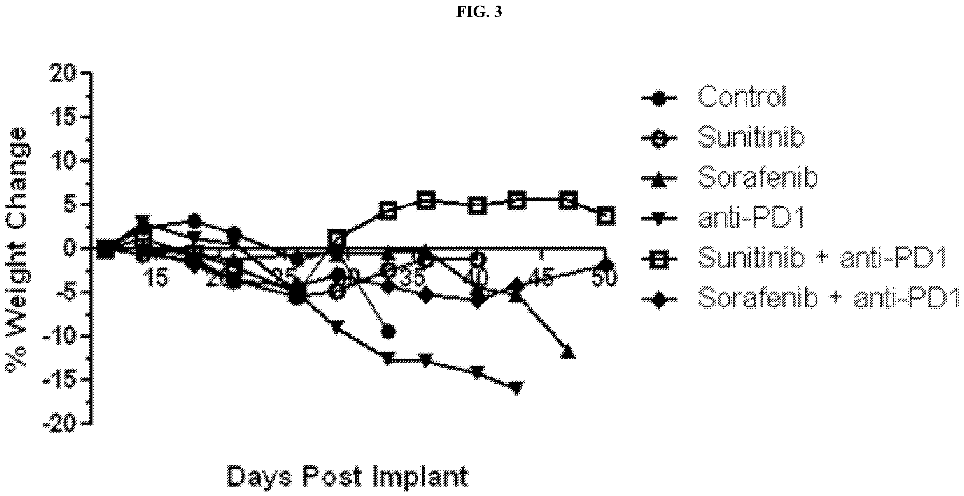

Figure 3 shows the effects of treatment with the following therapeutic agents on percentage change in body weight: control (filled circle), sunitinib (open circle), sorafenib (filled triangle), anti-PD-1 (filled inverted triangle), sunitinib + anti-PD-1 (open square), and sorafenib + anti-PD-1 (filled diamond).

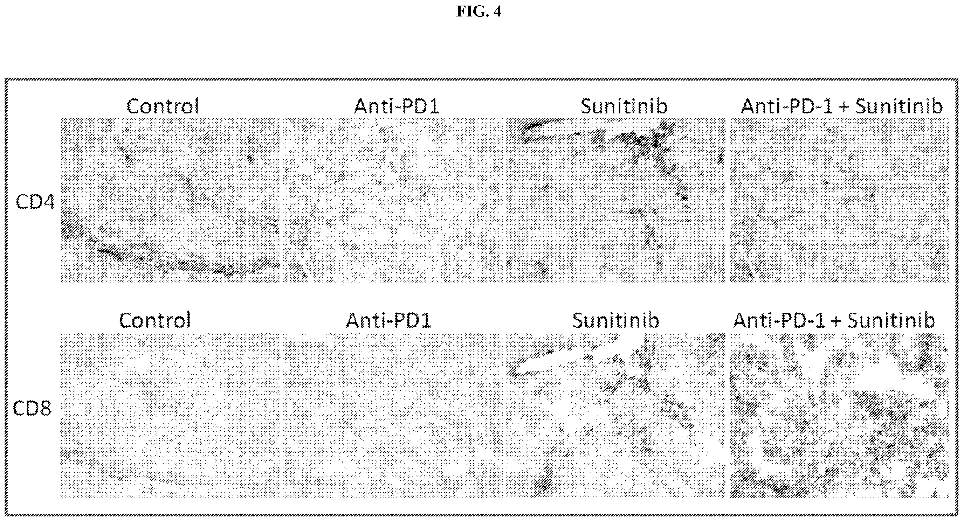

Figure 4 shows the infiltration of CD4⁺ and CD8⁺ T cells into Renca tumors post-treatment with control, anti-PD-1 alone, sunitinib alone, or anti-PD-1 in combination with sunitinib.

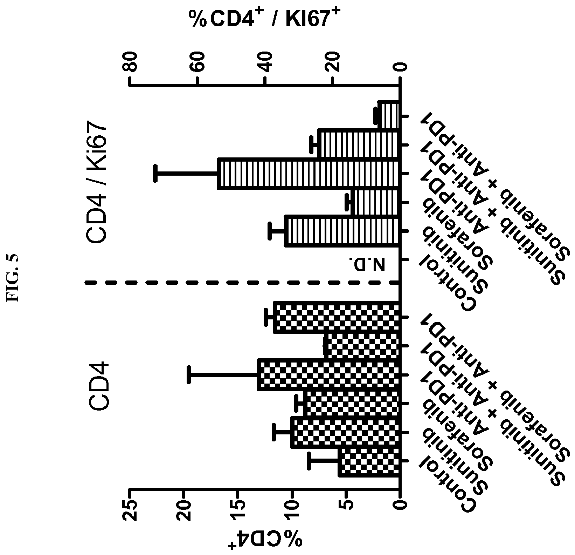

Figure 5 shows an assessment of the composition of tumor-infiltrating CD4⁺ immune cells by flow cytometry in a Renca tumor model following treatment with sunitinib, sorafenib or anti-PD-1 Ab alone, or anti-PD-1 Ab in combination with sunitinib or sorafenib.

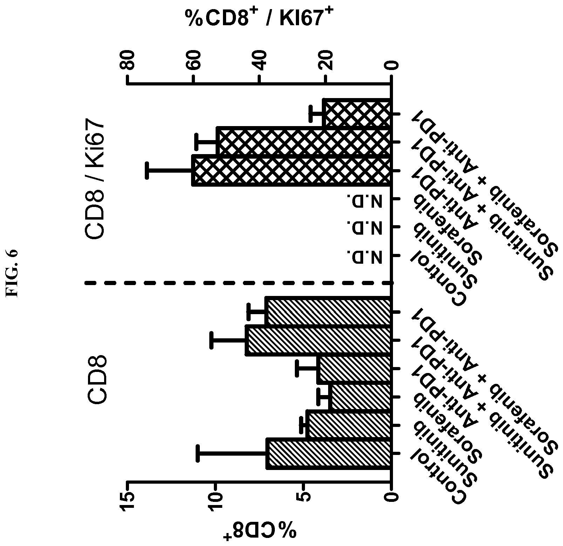

Figure 6 shows an assessment of the composition of tumor-infiltrating CD8⁺ immune cells by flow cytometry in a Renca tumor model following treatment with sunitinib, sorafenib or anti-PD-1 Ab alone, or anti-PD-1 Ab in combination with sunitinib or sorafenib.

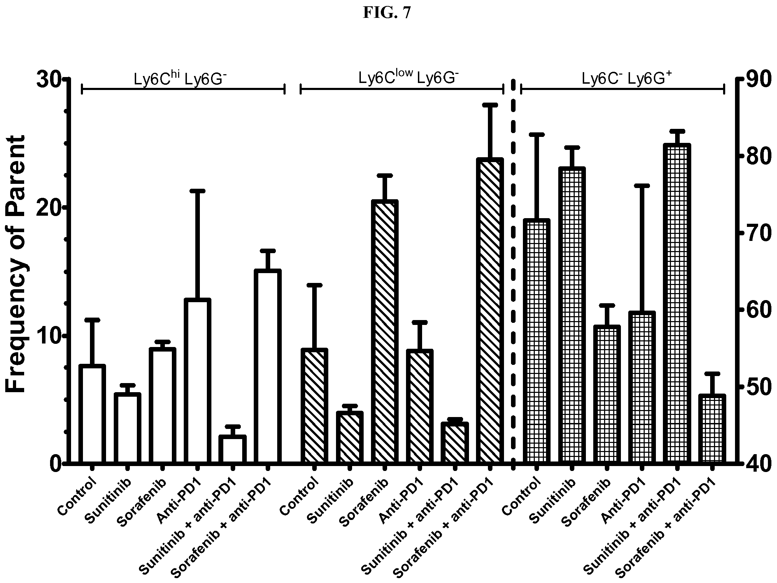

Figure 7 shows an assessment of myeloid derived tumor-infiltrating cell subsets (CD11b⁺/Ly6C^{hi}/Ly6G⁻, CD11b⁺/Ly6C^{low}/Ly6G⁻, or CD11b⁺/Ly6C⁻/Ly6G⁺) in a Renca tumor model following treatment with sunitinib, sorafenib or anti-PD-1 Ab alone, or anti-PD-1 Ab in combination with sunitinib or sorafenib. Gating: Cells → Singlets → Cd45⁺/Live → CD11b⁺.

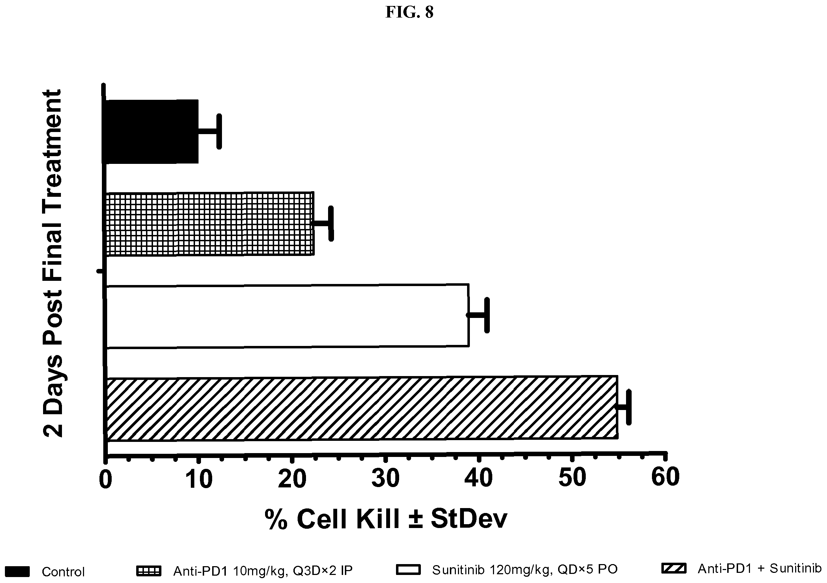

Figure 8 shows the effect of the combination of anti-PD-1 mAb and sunitinib on peptide-specific cytotoxic response in a CT26 tumor model. X axis shows the percentage of cell killing. Each bar represents from top to bottom (i) control, (ii) anti-PD-1 10 mg/kg, Q3D x 2 IP, (iii) sunitinib 120 mg/kg, QD x 5, PO, and (iv) anti-PD-1 + sunitinib.

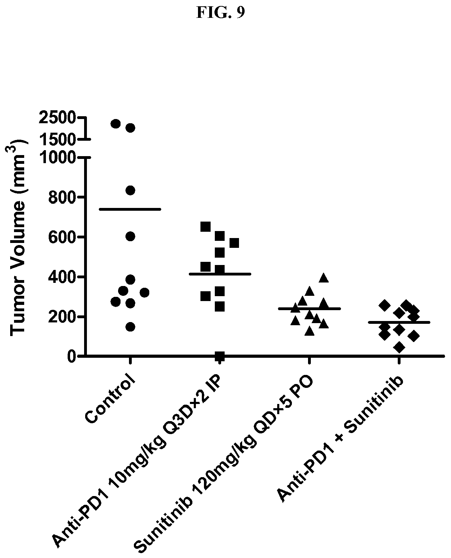

Figure 9 shows the effect of the combination of anti-PD-1 mAb and sunitinib on tumor volume in a CT26 tumor model. The tumor volume (mm3) is shown in the y-axis for administration of control, anti-PD-1 10 mg/kg Q3Dx IP, sunitinib 120mg/kg QDx5 PO, and anti-PD-1 + sunitinib.

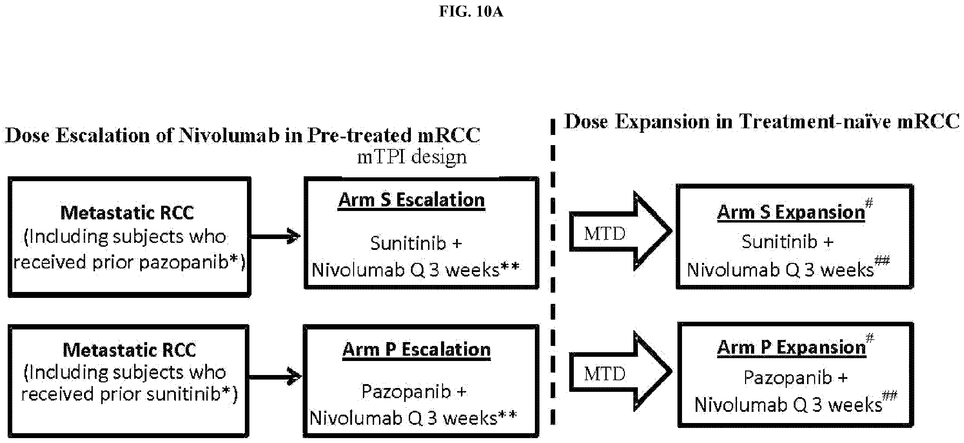

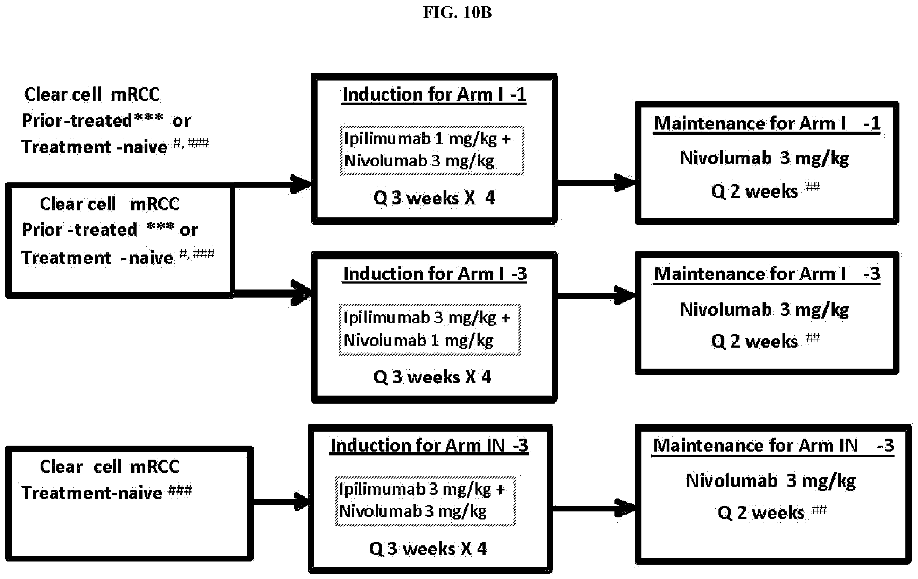

Figures 10A and 10B show the design of a Phase 1 study. Figures 10A and 10B show the study design of the combination of nivolumab and sunitinib or pazopanib (A), or nivolumab and ipilimumab (B), respectively, in subjects with advanced or metastatic RCC (mRCC) (NCT01472081). * Subjects who had received at least one prior systemic therapy regimen for advanced/metastatic RCC but had not received prior pazopanib or sunitinib were assigned in an alternating fashion to the two dose escalation arms when both arms were open. If only one arm was open, these subjects were assigned to the open arm. ** Until progressive disease (PD) or drug-related toxicity leading to discontinuation or subject withdrew consent. *** Subjects who had received at least one prior systemic therapy, and did not qualify for escalation-phase Arm S and/or P when they were open, were assigned in an alternating fashion to the two ipilimumab combination arms (1-1 and 1-3) when both arms were open. If only one arm were open, these subjects were assigned to the open arm. # Treatment-naive subjects were randomly assigned to one of the 4 arms (Expansion Arm S and P, and 1-1 and 1-3), if all arms were open, in a 1:1:1:1ratio until all enrollment was complete. ## Until progressive disease (PD) or drug-related toxicity leading to discontinuation or subject withdrew consent. Treatment beyond initial investigator-assessed RECIST 1.1-defined progression was considered in subjects experiencing investigator-assessed clinical benefit and tolerating study therapy. Such subjects were required to discontinue therapy when further progression was documented. ### For the 1-1 and 1-3 cohort expansion, and the IN-3 dose addition cohort, one prior adjuvant or neoadjuvant therapy for localized or locally advanced RCC was allowed provided recurrence occurred ≥ 6 months after the last dose of the adjuvant or neoadjuvant therapy. Prior cytokine based treatment for RCC [*e.g*., interferon-alpha (IFN-α) or interleukin 2 (IL-2)] as prior therapy was allowed. These minimally treated (including treatment naive) subjects were randomly assigned to one of these 3 arms (1-1, 1-3 and IN-3), if all arms were open, in a 1:1:1 ratio until all enrollment was complete.

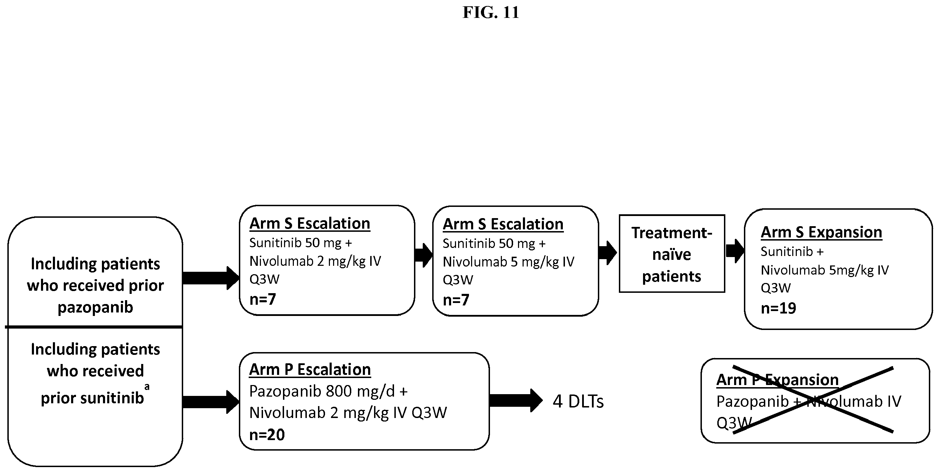

Figure 11 shows the dose escalation performed in the S and P arms of the nivolumab combination therapy study. The S arm comprised 7 pretreated subjects to whom were administered S + N2; 7 pretreated subjects to whom were administered S + N5; and 19 treatment-naive patients who entered the S + N5 expansion. The P arm comprised 20 pretreated subjects to whom were administered P + N2. There was no expansion of the P arm.

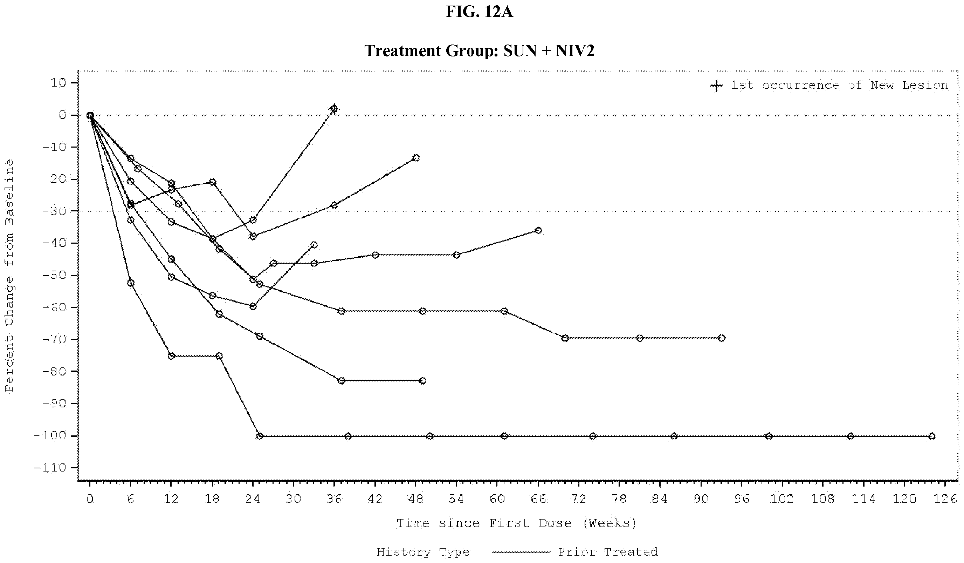

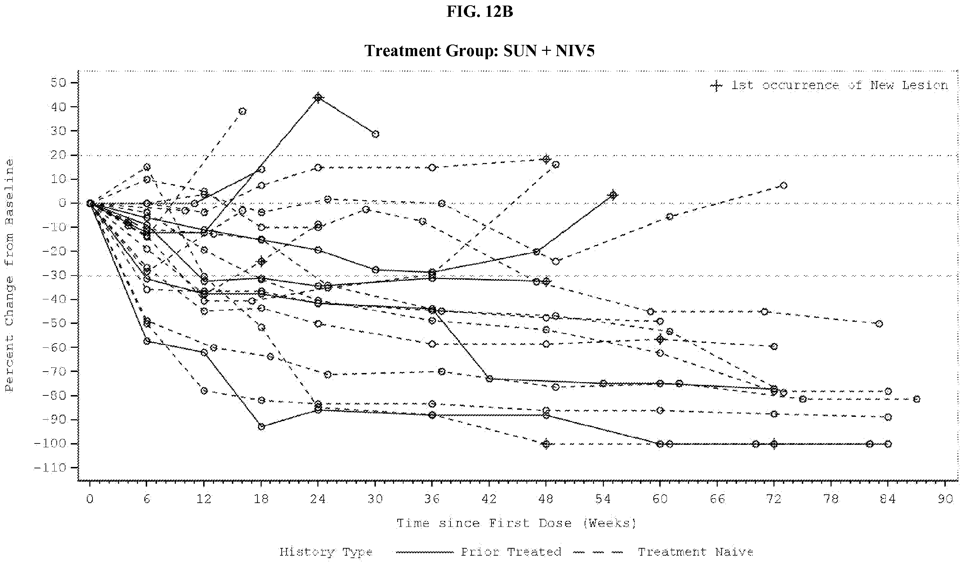

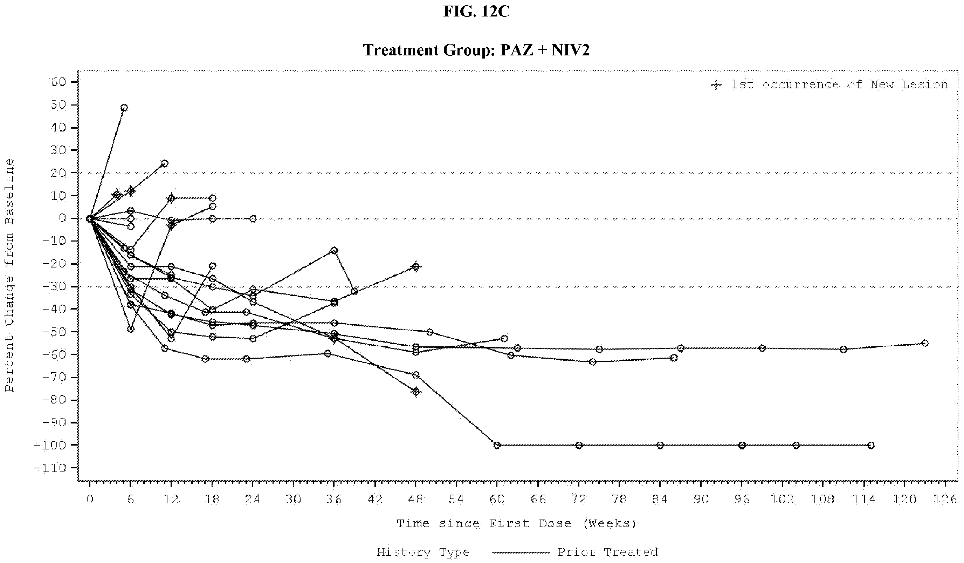

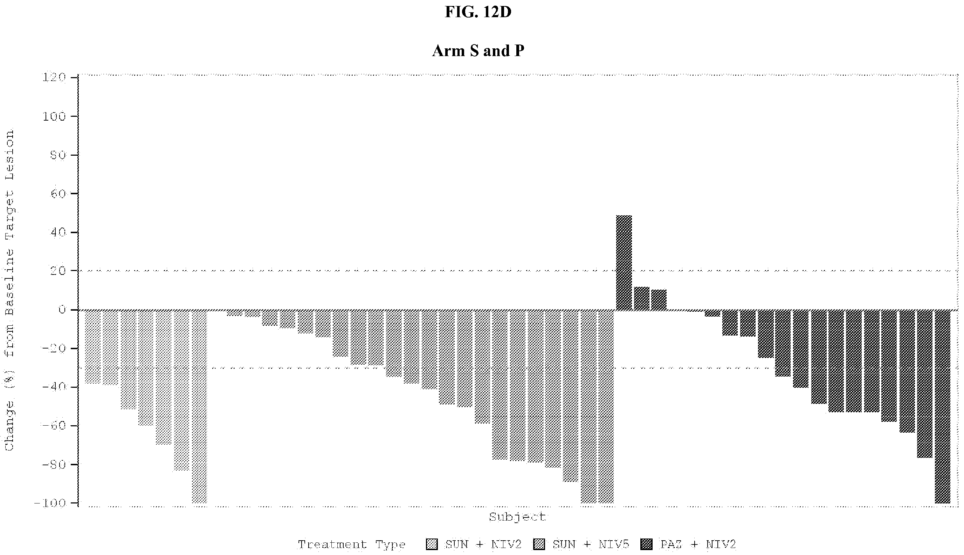

Figures 12A-D show clinical activity of RCC subjects treated with a combination of nivolumab and sunitinib or pazopanib. Spider plots show percentage changes from baseline in the tumor burden, measured as the sum of products of perpendicular diameters of all target lesions, in subjects who received various regimens. Figure 12A shows a regimen of sunitinib and 2 mg/kg nivolumab. Figure 12B shows a regimen of sunitinib and 5 mg/kg nivolumab. Figure 12C shows a regimen of pazopanib and 2 mg/kg nivolumab. "+" indicates the first occurrence of a new lesion. Figure 12D is a waterfall plot showing maximum percentage response in baseline target lesions in subjects in the S and P arms. Subjects with baseline target lesions and at least one post-baseline target lesion assessment are presented. Positive change in tumor burden indicates tumor growth; negative change in tumor burden indicates tumor reduction. In the spider and waterfall plots, horizontal lines denote 30% decrease (RECIST 1.1 threshold for PR) and 20% increase (RECIST 1.1 threshold for PD). Not all reductions of 30% or greater from baseline are PRs.

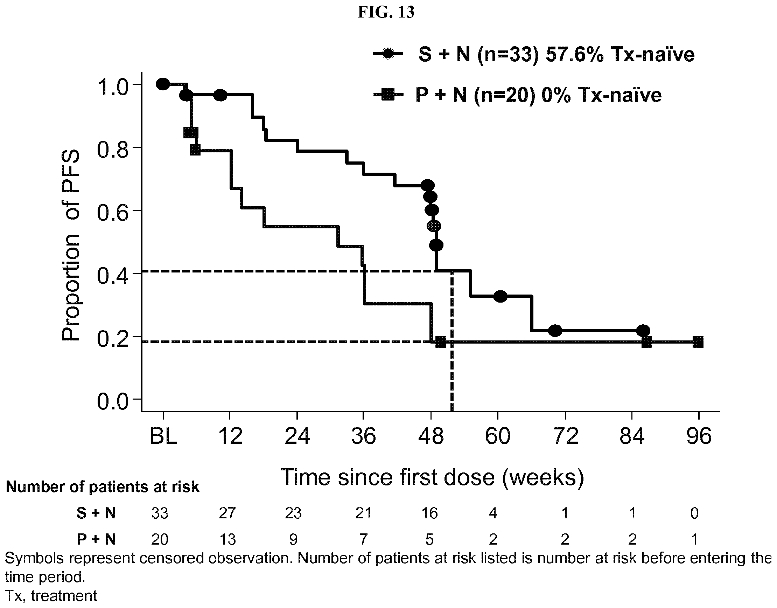

Figure 13 shows the proportion of progression-free survival (PFS) plotted against time for subjects treated with nivolumab and sunitinib (S + N) or nivolumab and pazopanib (P + N). Symbols represent censored observation. The number of patients at risk listed is the number at risk before entering the time period. Tx: treatment.

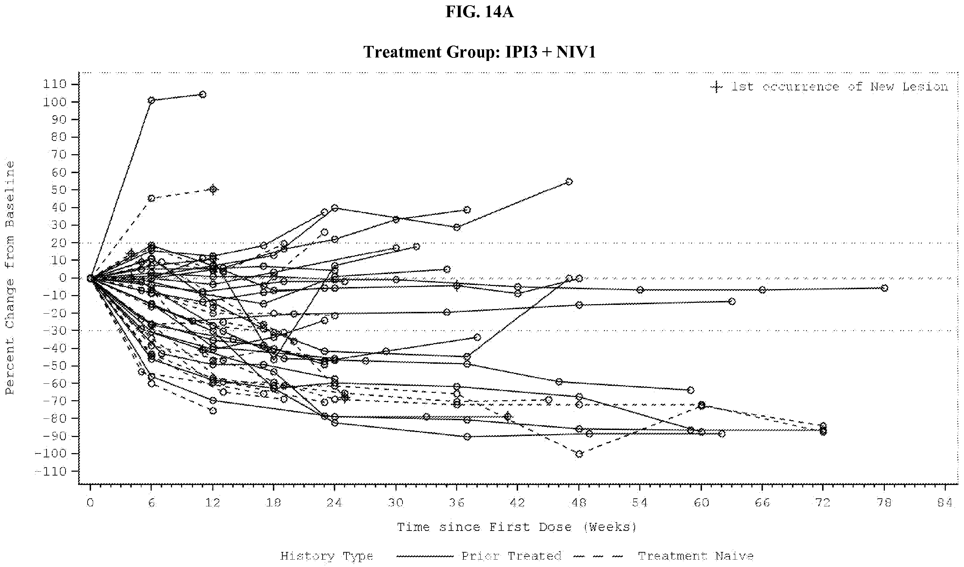

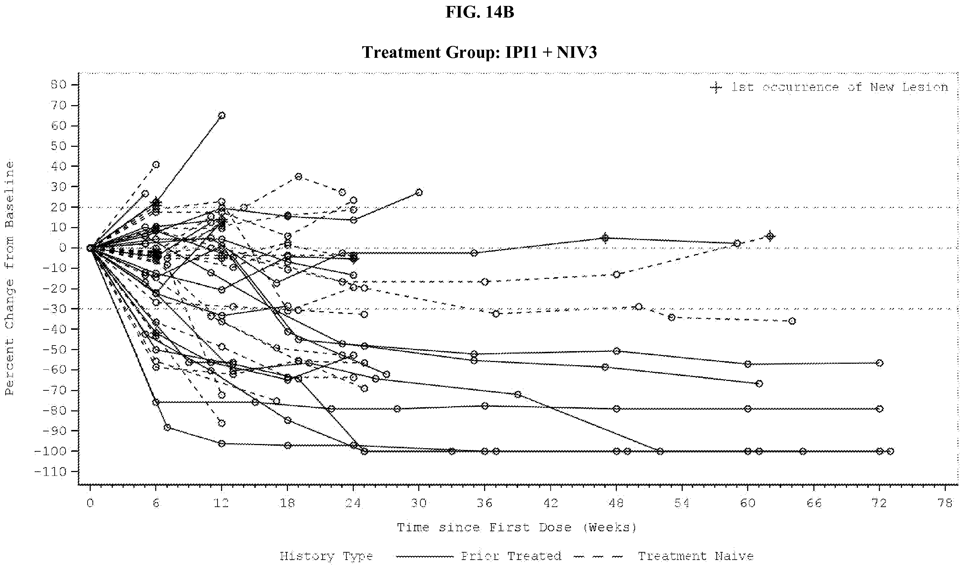

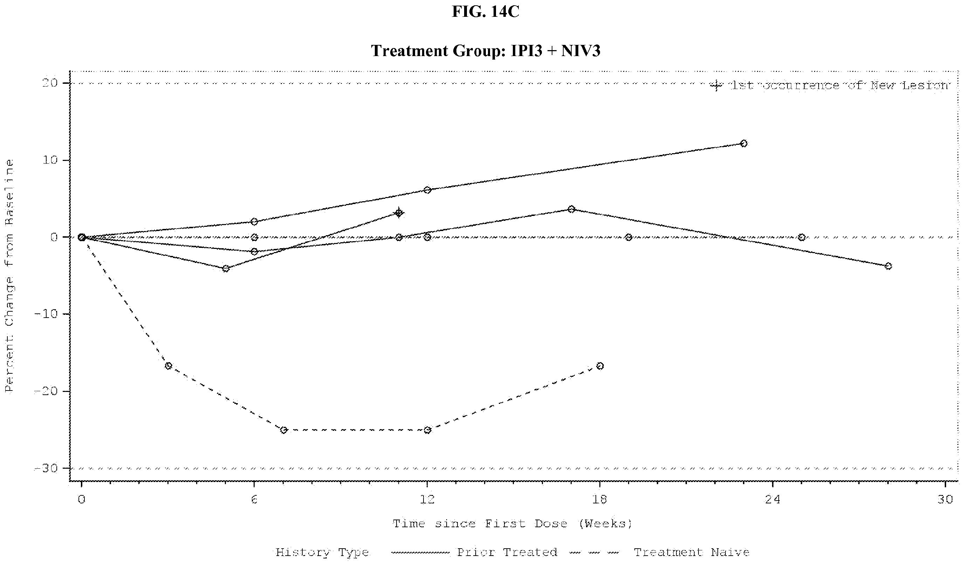

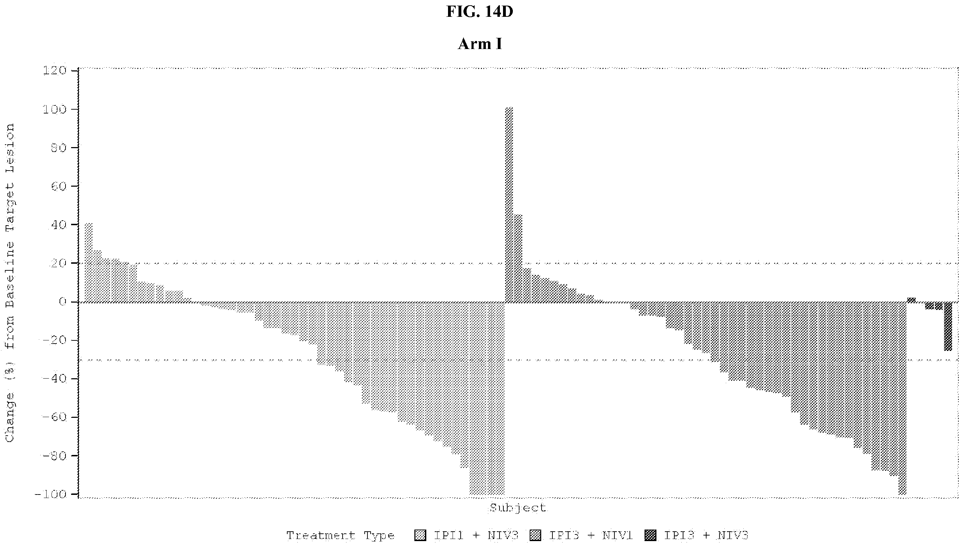

Figures 14A-14D show clinical activity of RCC subjects treated with a combination of ipilimumab and nivolumab. Spider plots show percentage changes from baseline in the tumor burden, measured as the sum of products of perpendicular diameters of all target lesions, in subjects who received various regimens. Figure 14A shows a regimen of 3 mg/kg ipilimumab and 1 mg/kg nivolumab. Figure 14B shows a regimen of 1 mg/kg ipilimumab and 3 mg/kg nivolumab. Figure 14C shows a regimen of 3 mg/kg ipilimumab and 3 mg/kg nivolumab. "+" indicates the first occurrence of a new lesion. Figure 14D is a waterfall plot showing maximum percentage response in baseline target lesions in subjects in the 1-1, 1-3, and IN-3 arms. Subjects with baseline target lesions and at least one post-baseline target lesion assessment are presented. Positive change in tumor burden indicates tumor growth; negative change in tumor burden indicates tumor reduction. In the spider and waterfall plots, horizontal lines denote 30% decrease (RECIST 1.1 threshold for PR) and 20% increase (RECIST 1.1 threshold for PD). Not all reductions of 30% or greater from baseline are PRs.

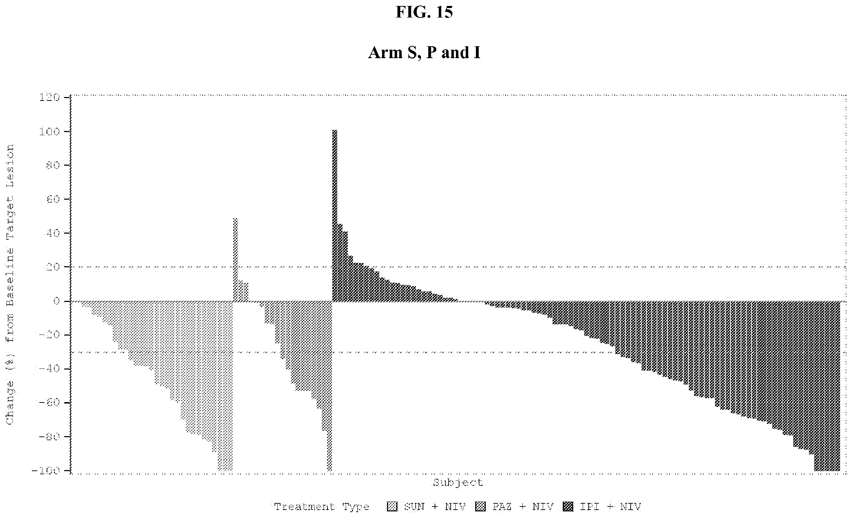

Figure 15 shows a comparison of waterfall plots for subjects in the S, P and I arms. Subjects with baseline target lesions and at least one post-baseline target lesion assessment are presented. Positive change in tumor burden indicates tumor growth; negative change in tumor burden indicates tumor reduction. Horizontal lines denote 30% decrease (RECIST 1.1 threshold for PR) and 20% increase (RECIST 1.1 threshold for PD). Not all reductions of 30% or greater from baseline are PRs.

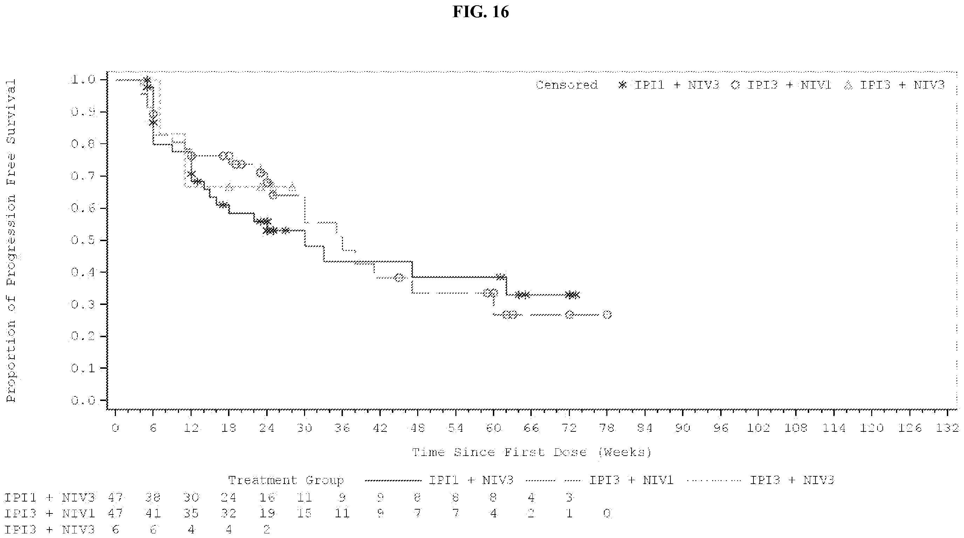

Figure 16 shows the proportion of PFS plotted against time since first dose (weeks) for subjects treated with 1 mg/kg ipilimumab + 3 mg/kg nivolumab (IPI1+INV3) (asterisk), with 3 mg/kg ipilimumab + 1 mg/kg nivolumab (IPI3 + NIV1)(circle), and 3 mg/kg nivolumab + 3 mg/kg ipilimumab (IPI3 + NIV3)(triangle). Symbols represent censored observation. The number of patients at risk listed is the number at risk before entering the time period.

### DETAILED DESCRIPTION OF THE INVENTION

The present invention relates to methods for treating a renal cancer patient comprising administering to the patient a combination of an anti-PD-1 Ab or an antigen-binding fragment thereof and another anti-cancer agent. In certain embodiments, the other anti-cancer agent is an anti-CTLA-4 Ab, an anti-angiogenic TKI, or any combination thereof.

### Terms

In order that the present disclosure may be more readily understood, certain terms are first defined. As used in this application, except as otherwise expressly provided herein, each of the following terms shall have the meaning set forth below. Additional definitions arc set forth throughout the application.

"Administering" refers to the physical introduction of a composition comprising a therapeutic agent to a subject, using any of the various methods and delivery systems known to those skilled in the art. Preferred routes of administration for the anti-PD-1 Ab include intravenous, intramuscular, subcutaneous, intraperitoneal, spinal or other parenteral routes of administration, for example by injection or infusion. The phrase "parenteral administration" as used herein means modes of administration other than enteral and topical administration, usually by injection, and includes, without limitation, intravenous, intramuscular, intraarterial, intrathecal, intralymphatic, intralesional, intracapsular, intraorbital, intracardiac, intradermal, intraperitoneal, transtracheal, subcutaneous, subcuticular, intraarticular, subcapsular, subarachnoid, intraspinal, epidural and intrasternal injection and infusion, as well as *in vivo* electroporation. The TKI is typically administered via a non-parenteral route, preferably orally. Other non-parenteral routes include a topical, epidermal or mucosal route of administration, for example, intranasally, vaginally, rectally, sublingually or topically. Administering can also be performed, for example, once, a plurality of times, and/or over one or more extended periods.

An "adverse event" (AE) as used herein is any unfavorable and generally unintended or undesirable sign (including an abnormal laboratory finding), symptom, or disease associated with the use of a medical treatment. For example, an adverse event may be associated with activation of the immune system or expansion of immune system cells (*e.g.,* T cells) in response to a treatment. A medical treatment may have one or more associated AEs and each AE may have the same or different level of severity. Reference to methods capable of "altering adverse events" means a treatment regime that decreases the incidence and/or severity of one or more AEs associated with the use of a different treatment regime.

An "antibody" (Ab) shall include, without limitation, a glycoprotein immunoglobulin which binds specifically to an antigen and comprises at least two heavy (H) chains and two light (L) chains interconnected by disulfide bonds, or an antigen-binding portion thereof. Each H chain comprises a heavy chain variable region (abbreviated herein as V*_{H}*) and a heavy chain constant region. The heavy chain constant region comprises three constant domains, C_{*H*1}, C_{*H*2} and C_{*H*3}*.* Each light chain comprises a light chain variable region (abbreviated herein as V*_{L}*) and a light chain constant region. The light chain constant region is comprises one constant domain, C*_{L}*. The V*_{H}* and V*_{L}* regions can be further subdivided into regions of hypervariability, termed complementarity determining regions (CDRs), interspersed with regions that are more conserved, termed framework regions (FR). Each V*_{H}* and V*_{L}* comprises three CDRs and four FRs, arranged from amino-terminus to carboxy-terminus in the following order: FR1, CDR1, FR2, CDR2, FR3, CDR3, FR4. The variable regions of the heavy and light chains contain a binding domain that interacts with an antigen. The constant regions of the Abs may mediate the binding of the immunoglobulin to host tissues or factors, including various cells of the immune system (*e.g*., effector cells) and the first component (C1q) of the classical complement system.

An immunoglobulin may derive from any of the commonly known isotypes, including but not limited to IgA, secretory IgA, IgG and IgM. IgG subclasses are also well known to those in the art and include but are not limited to human IgG1, IgG2, IgG3 and IgG4. "Isotype" refers to the Ab class or subclass (*e.g*., IgM or IgG1) that is encoded by the heavy chain constant region genes. The term "antibody" includes, by way of example, both naturally occurring and non-naturally occurring Abs; monoclonal and polyclonal Abs; chimeric and humanized Abs; human or nonhuman Abs; wholly synthetic Abs; and single chain Abs. A nonhuman Ab may be humanized by recombinant methods to reduce its immunogenicity in man. Where not expressly stated, and unless the context indicates otherwise, the term "antibody" also includes an antigen-binding fragment or an antigen-binding portion of any of the aforementioned immunoglobulins, and includes a monovalent and a divalent fragment or portion, and a single chain Ab.

An "isolated antibody" refers to an Ab that is substantially free of other Abs having different antigenic specificities (*e.g*., an isolated Ab that binds specifically to PD-1 is substantially free of Abs that bind specifically to antigens other than PD-1). An isolated Ab that binds specifically to PD-1 may, however, have cross-reactivity to other antigens, such as PD-1 molecules from different species. Moreover, an isolated Ab may be substantially free of other cellular material and/or chemicals.

The term "monoclonal antibody" ("mAb") refers to a non-naturally occurring preparation of Ab molecules of single molecular composition, *i.e.,* Ab molecules whose primary sequences are essentially identical, and which exhibits a single binding specificity and affinity for a particular epitope. A mAb is an example of an isolated Ab. MAbs may be produced by hybridoma, recombinant, transgenic or other techniques known to those skilled in the art.

A "human" antibody (HuMAb) refers to an Ab having variable regions in which both the framework and CDR regions are derived from human germline immunoglobulin sequences. Furthermore, if the Ab contains a constant region, the constant region also is derived from human germline immunoglobulin sequences. The human Abs of the invention may include amino acid residues not encoded by human germline immunoglobulin sequences (*e.g*., mutations introduced by random or site-specific mutagenesis *in vitro* or by somatic mutation *in vivo*)*.* However, the term "human antibody," as used herein, is not intended to include Abs in which CDR sequences derived from the germline of another mammalian species, such as a mouse, have been grafted onto human framework sequences. The terms "human" Abs and "fully human" Abs and are used synonymously.

A "humanized antibody" refers to an Ab in which some, most or all of the amino acids outside the CDR domains of a non-human Ab are replaced with corresponding amino acids derived from human immunoglobulins. In one embodiment of a humanized form of an Ab, some, most or all of the amino acids outside the CDR domains have been replaced with amino acids from human immunoglobulins, whereas some, most or all amino acids within one or more CDR regions are unchanged. Small additions, deletions, insertions, substitutions or modifications of amino acids are permissible as long as they do not abrogate the ability of the Ab to bind to a particular antigen. A "humanized" Ab retains an antigenic specificity similar to that of the original Ab.

A "chimeric antibody" refers to an Ab in which the variable regions are derived from one species and the constant regions are derived from another species, such as an Ab in which the variable regions are derived from a mouse Ab and the constant regions are derived from a human Ab.

An "anti-antigen" Ab refers to an Ab that binds specifically to the antigen. For example, an anti-PD-1 Ab binds specifically to PD-1 and an anti-CTLA-4 Ab binds specifically to CTLA-4.

An "antigen-binding portion" of an Ab (also called an "antigen-binding fragment") refers to one or more fragments of an Ab that retain the ability to bind specifically to the antigen bound by the whole Ab.

A "cancer" refers a broad group of various diseases characterized by the uncontrolled growth of abnormal cells in the body. Unregulated cell division and growth divide and grow results in the formation of malignant tumors that invade neighboring tissues and may also metastasize to distant parts of the body through the lymphatic system or bloodstream. A "cancer" or "cancer tissue" can include a tumor.

"Cytotoxic T-Lymphocyte Antigen-4 (CTLA-4) refers to an immunoinhibitory receptor belonging to the CD28 family. CTLA-4 is expressed exclusively on T cells *in vivo,* and binds to two ligands, CD80 and CD86 (also called B7-1 and B7-2, respectively). The term "CTLA-4" as used herein includes human CTLA-4 (hCTLA-4), variants, isoforms, and species homologs of hCTLA-4, and analogs having at least one common epitope with hCTLA-4. The complete hCTLA-4 sequence can be found under GenBank Accession No. AAB59385.

The term "immunotherapy" refers to the treatment of a subject afflicted with, or at risk of contracting or suffering a recurrence of, a disease by a method comprising inducing, enhancing, suppressing or otherwise modifying an immune response.

"Treatment" or "therapy" of a subject refers to any type of intervention or process performed on, or the administration of an active agent to, the subject with the objective of reversing, alleviating, ameliorating, inhibiting, slowing down or preventing the onset, progression, development, severity or recurrence of a symptom, complication or condition, or biochemical indicia associated with a disease.

"Programmed Death-1 (PD-1)" refers to an immunoinhibitory receptor belonging to the CD28 family. PD-1 is expressed predominantly on previously activated T cells *in vivo,* and binds to two ligands, PD-L1 and PD-L2. The term "PD-1" as used herein includes human PD-1 (hPD-1), variants, isoforms, and species homologs of hPD-1, and analogs having at least one common epitope with hPD-1. The complete hPD-1 sequence can be found under GenBank Accession No. U64863.

"Programmed Death Ligand-1 (PD-L1)" is one of two cell surface glycoprotein ligands for PD-1 (the other being PD-L2) that downregulate T cell activation and cytokine secretion upon binding to PD-1. The term "PD-L1" as used herein includes human PD-L1 (hPD-L1), variants, isoforms, and species homologs of hPD-L1, and analogs having at least one common epitope with hPD-L1. The complete hPD-L1 sequence can be found under GenBank Accession No. Q9NZQ7.

A "subject" includes any human or nonhuman animal. The term "nonhuman animal" includes, but is not limited to, vertebrates such as nonhuman primates, sheep, dogs, and rodents such as mice, rats and guinea pigs. In some embodiments, the subject is a human. The terms, "subject" and "patient" are used interchangeably herein.

A "therapeutically effective amount" or "therapeutically effective dosage" of a drug or therapeutic agent is any amount of the drug that, when used alone or in combination with another therapeutic agent, protects a subject against the onset of a disease or promotes disease regression evidenced by a decrease in severity of disease symptoms, an increase in frequency and duration of disease symptom-free periods, or a prevention of impairment or disability due to the disease affliction. The ability of a therapeutic agent to promote disease regression can be evaluated using a variety of methods known to the skilled practitioner, such as in human subjects during clinical trials, in animal model systems predictive of efficacy in humans, or by assaying the activity of the agent in *in vitro* assays.

As used herein, the term "subtherapeutic dose" or "subtherapeutically effective amount" means a dose of a therapeutic compound (e.g., an antibody) that is lower than the usual or typical dose of the therapeutic compound when administered alone for the treatment of a hyperproliferative disease (e.g., cancer). For example, a subtherapeutic dose of an anti-PDl antibody (nivolumab) is a single dose of the antibody at less than about 3 mg/kg.

The use of the alternative (*e.g*., "or") should be understood to mean either one, both, or any combination thereof of the alternatives. As used herein, the indefinite articles "a" or "an" should be understood to refer to "one or more" of any recited or enumerated component.

By way of example, an "anti-cancer agent" promotes cancer regression in a subject. In other embodiments, a therapeutically effective amount of the drug promotes cancer regression to the point of eliminating the cancer. "Promoting cancer regression" means that administering an effective amount of the drug, alone or in combination with an anti-neoplastic agent, results in a reduction in tumor growth or size, necrosis of the tumor, a decrease in severity of at least one disease symptom, an increase in frequency and duration of disease symptom-free periods, or a prevention of impairment or disability due to the disease affliction. In addition, the terms "effective" and "effectiveness" with regard to a treatment includes both pharmacological effectiveness and physiological safety. Pharmacological effectiveness refers to the ability of the drug to promote cancer regression in the patient. Physiological safety refers to the level of toxicity, or other adverse physiological effects at the cellular, organ and/or organism level (adverse effects) resulting from administration of the drug.

By way of example for the treatment of tumors, a therapeutically effective amount of an anti-cancer agent preferably inhibits cell growth or tumor growth by at least about 20%, more preferably by at least about 40%, even more preferably by at least about 60%, and still more preferably by at least about 80% relative to untreated subjects. In other embodiments of the invention, tumor regression may be observed and continue for a period of at least about 20 days, more preferably at least about 40 days, or even more preferably at least about 60 days. Notwithstanding these ultimate measurements of therapeutic effectiveness, evaluation of immunotherapeutic drugs must also make allowance for "immune-related" response patterns.

An "immune-related" response pattern refers to a clinical response pattern often observed in cancer patients treated with immunotherapeutic agents that produce antitumor effects by inducing cancer-specific immune responses or by modifying native immune processes. This response pattern is characterized by a beneficial therapeutic effect that follows an initial increase in tumor burden or the appearance of new lesions, which in the evaluation of traditional chemotherapeutic agents would be classified as disease progression and would be synonymous with drug failure. Accordingly, proper evaluation of immunotherapeutic agents may require long-term monitoring of the effects of these agents on the target disease.

A therapeutically effective amount of a drug includes a "prophylactically effective amount," which is any amount of the drug that, when administered alone or in combination with an anti-neoplastic agent to a subject at risk of developing a cancer (*e.g*., a subject having a pre-malignant condition) or of suffering a recurrence of cancer, inhibits the development or recurrence of the cancer. In embodiments, the prophylactically effective amount prevents the development or recurrence of the cancer entirely. "Inhibiting" the development or recurrence of a cancer means either lessening the likelihood of the cancer's development or recurrence, or preventing the development or recurrence of the cancer entirely.

The use of the alternative (*e.g.*, "or") should be understood to mean either one, both, or any combination thereof of the alternatives. As used herein, the indefinite articles "a" or "an" should be understood to refer to "one or more" of any recited or enumerated component.

The terms "about" or "comprising essentially of" refer to a value or composition that is within an acceptable error range for the particular value or composition as determined by one of ordinary skill in the art, which will depend in part on how the value or composition is measured or determined, *i.e.,* the limitations of the measurement system. For example, "about" or "comprising essentially of" can mean within 1 or more than 1 standard deviation per the practice in the art. Alternatively, "about" or "comprising essentially of" can mean a range of up to 10% or 20% (i.e., ±10% or ±20%). For example, about 3mg/kg can include any number between 2.7 mg/kg and 3.3 mg/kg (for 10%) or between 2.4 mg/kg and 3.6 mg/kg (for 20%). Furthermore, particularly with respect to biological systems or processes, the terms can mean up to an order of magnitude or up to 5-fold of a value. When particular values or compositions are provided in the application and claims, unless otherwise stated, the meaning of "about" or "comprising essentially of" should be assumed to be within an acceptable error range for that particular value or composition.

As described herein, any concentration range, percentage range, ratio range or integer range is to be understood to include the value of any integer within the recited range and, when appropriate, fractions thereof (such as one tenth and one hundredth of an integer), unless otherwise indicated.

Various aspects of the invention are described in further detail in the following subsections.

### Anti-PD-1 antibodies

PD-1 is a key immune checkpoint receptor expressed by activated T and B cells and mediates immunosuppression. PD-1 is a member of the CD28 family of receptors, which includes CD28, CTLA-4, ICOS, PD-1, and BTLA. Two cell surface glycoprotein ligands for PD-1 have been identified, Programmed Death Ligand-1 (PD-L1) and Programmed Death Ligand-2 (PD-L2), that are expressed on antigen-presenting cells as well as many human cancers and have been shown to down regulate T cell activation and cytokine secretion upon binding to PD-1. Inhibition of the PD-1/PD-L1 interaction mediates potent antitumor activity in preclinical models.

HuMAbs that bind specifically to PD-1 with high affinity have been disclosed in U.S. Patent Nos. 8,008,449 and 8,779,105. Other anti-PD-1 mAbs have been described in, for example, U.S. Patent Nos. 6,808,710, 7,488,802, 8,168,757 and 8,354,509, and PCT Publication No. WO 2012/145493. Each of the anti-PD-1 HuMAbs disclosed in U.S. Patent No. 8,008,449 has been demonstrated to exhibit one or more of the following characteristics: (a) binds to human PD-1 with a K_{D} of 1 x 10⁻⁷ M or less, as determined by surface plasmon resonance using a Biacore biosensor system; (b) does not substantially bind to human CD28, CTLA-4 or ICOS; (c) increases T-cell proliferation in a Mixed Lymphocyte Reaction (MLR) assay; (d) increases interferon-γ production in an MLR assay; (e) increases IL-2 secretion in an MLR assay; (f) binds to human PD-1 and cynomolgus monkey PD-1; (g) inhibits the binding of PD-L1 and/or PD-L2 to PD-1; (h) stimulates antigen-specific memory responses; (i) stimulates Ab responses; and (j) inhibits tumor cell growth *in vivo.* Anti-PD-1 Abs usable in the present invention include mAbs that bind specifically to human PD-1 and exhibit at least one, preferably at least five, of the preceding characteristics.

In one embodiment, the anti-PD-1 Ab is nivolumab. Nivolumab (also known as "OPDIVO®"; formerly designated 5C4, BMS-936558, MDX-1106, or ONO-4538) is a fully human IgG4 (S228P) PD-1 immune checkpoint inhibitor Ab that selectively prevents interaction with PD-1 ligands (PD-L1 and PD-L2), thereby blocking the down-regulation of antitumor T-cell functions (U.S. Patent No. 8,008,449; Wang et al., 2014 Cancer Immunol Res. 2(9):846-56).

In another embodiment, the anti-PD-1 Ab is pembrolizumab. Pembrolizumab (also known as "KEYTRUDA®", lambrolizumab, and MK-3475) is a humanized monoclonal IgG4 antibody directed against human cell surface receptor PD-1 (programmed death-1 or programmed cell death-1). Pembrolizumab is described, for example, in U.S. Patent Nos. 8,354,509 and 8,900,587; *see also* http://www.cancer.gov/drugdictionary?cdrid=695789 (last accessed: December 14, 2014). Pembrolizumab has been approved by the FDA for the treatment of relapsed or refractory melanoma.

In other embodiments, the anti-PD-1 Ab is MEDI0608 (formerly AMP-514), which is a monoclonal antibody. MEDI0608 is described, for example, in US Pat. No. 8,609,089B2 or in http://www.cancer.gov/drugdictionary?cdrid=756047 (last accessed December 14, 2014).

In some embodiments, the anti-PD-1 antibody is Pidilizumab (CT-011), which is a humanized monoclonal antibody. Pidilizumab is described in US Pat. No. 8,686,119 B2 or WO 2013/014668 A1.

Anti-PD-1 Abs usable in the disclosed methods also include isolated Abs that bind specifically to human PD-1 and cross-compete for binding to human PD-1 with nivolumab (*see, e.g.,* U.S. Patent Nos. 8,008,449 and 8,779,105; WO 2013/173223). The ability of Abs to cross-compete for binding to an antigen indicates that these Abs bind to the same epitope region of the antigen and sterically hinder the binding of other cross-competing Abs to that particular epitope region. These cross-competing Abs are expected to have functional properties very similar those of nivolumab by virtue of their binding to the same epitope region of PD-1. Cross-competing Abs can be readily identified based on their ability to cross-compete with nivolumab in standard PD-1 binding assays such as Biacore analysis, ELISA assays or flow cytometry (*see, e.g.,* WO 2013/173223).

In certain embodiments, the Abs that cross-compete for binding to human PD-1 with, or bind to the same epitope region of human PD-1 as, nivolumab are mAbs. For administration to human subjects, these cross-competing Abs are preferably chimeric Abs, or more preferably humanized or human Abs. Such chimeric, humanized or human mAbs can be prepared and isolated by methods well known in the art.

Anti-PD-1 Abs usable in the methods of the disclosed invention also include antigen-binding portions of the above Abs. It has been amply demonstrated that the antigen-binding function of an Ab can be performed by fragments of a full-length Ab. Examples of binding fragments encompassed within the term "antigen-binding portion" of an Ab include (i) a Fab fragment, a monovalent fragment consisting of the V*_{L}*, V*_{H},* C*_{L}* and C_{*H*1} domains; (ii) a F(ab')₂ fragment, a bivalent fragment comprising two Fab fragments linked by a disulfide bridge at the hinge region; (iii) a Fd fragment consisting of the V*_{H}* and C_{*H*1} domains; and (iv) a Fv fragment consisting of the V*_{L}* and V*_{H}* domains of a single arm of an Ab.

### Anti-CTLA-4 antibodies

Anti-CTLA-4 antibodies of the instant invention bind to human CTLA-4 so as to disrupt the interaction of CTLA-4 with a human B7 receptor. Because the interaction of CTLA-4 with B7 transduces a signal leading to inactivation of T-cells bearing the CTLA-4 receptor, disruption of the interaction effectively induces, enhances or prolongs the activation of such T cells, thereby inducing, enhancing or prolonging an immune response.

HuMAbs that bind specifically to CTLA-4 with high affinity have been disclosed in U.S. Patent Nos. 6,984,720 and 7,605,238. Other anti-PD-1 mAbs have been described in, for example, U.S. Patent Nos. 5,977,318, 6,051,227, 6,682,736, and 7,034,121. The anti-PD-1 HuMAbs disclosed in U.S. Patent No. Nos. 6,984,720 and 7,605,238 have been demonstrated to exhibit one or more of the following characteristics: (a) binds specifically to human CTLA-4 with a binding affinity reflected by an equilibrium association constant (K*ₐ*) of at least about 10⁷ M⁻¹, or about 10⁹ M⁻¹, or about 10¹⁰ M⁻¹ to 10¹¹ M⁻¹ or higher, as determined by Biacore analysis; (b) a kinetic association constant (k*ₐ*) of at least about 10³, about 10⁴, or about 10⁵ m⁻¹ s⁻¹; (c) a kinetic disassociation constant (k*_{d}*) of at least about 10³, about 10⁴, or about 10⁵ m⁻¹ s⁻¹; and (d) inhibits the binding of CTLA-4 to B7-1 (CD80) and B7-2 (CD86). Anti-CTLA-4 Abs usable in the present invention include mAbs that bind specifically to human CTLA-4 and exhibit at least one, and preferably at least three of the preceding characteristics. An exemplary clinical anti-CTLA-4 Ab is the human mAb 10D1 (now known as ipilimumab and marketed as YERVOY®) as disclosed in U.S. Patent No. 6,984,720.

An exemplary clinical anti-CTLA-4 Ab is the human mAb 10D1 (now known as ipilimumab and marketed as YERVOY®) as disclosed in U.S. Patent No. 6,984,720. Ipilimumab is an anti-CTLA-4 Ab for use in the methods disclosed herein. Ipilimumab is a fully human, IgG1 monoclonal Ab that blocks the binding of CTLA-4 to its B7 ligands, thereby stimulating T cell activation and improving overall survival (OS) in patients with advanced melanoma.

Another anti-CTLA-4 Ab usable in the present methods is tremelimumab (also known as CP-675,206). Tremelimumab is human IgG2 monoclonal anti-CTLA-4 antibody. Tremelimumab is described in WO/2012/122444, U.S. Publ. No. 2012/263677, or WO Publ. No. 2007/113648 A2.

Anti-CTLA-4 Abs usable in the disclosed methods also include isolated Abs that bind specifically to human PD-1 and cross-compete for binding to human CTLA-4 with ipilimumab or tremelimumab or bind to the same epitope region of human CTLA-4 as . In certain embodiments, the Abs that cross-compete for binding to human CTLA-4 with, or bind to the same epitope region of human PD-1 as does ipilimumab or tremelimumab, are Abs comprising a heavy chain of the human IgG1 isotype. For administration to human subjects, these cross-competing Abs are preferably chimeric Abs, or more preferably humanized or human Abs. Usable anti-CTLA-4 Abs also include antigen-binding portions of the above Abs such as Fab, F(ab')₂, Fd or Fv fragments.

### Standard-of-Care Therapies for Renal Cancer

The present invention also includes a combination therapy of an anti-PD-1 antibody with a standard of care therapy for treatment of renal cancer. The standard of care therapy can be performed on the subject any time before, during, or after the anti-PD1 antibody treatment. In some cases, the standard-of-care therapy and PD-1 antibody treatment are also combined with an additional treatment. Standard-of-care therapies for different types of cancer are well known by persons of skill in the art. For example, the National Comprehensive Cancer Network (NCCN), an alliance of 21 major cancer centers in the USA, publishes the NCCN Clinical Practice Guidelines in Oncology (NCCN GUIDELINES®) that provide detailed up-to-date information on the standard-of-care treatments for a wide variety of cancers (*see* NCCN GUIDELINES®, 2014).

RCC is the most common type of kidney cancer in adults, responsible for approximately 90% of renal tumors, and 80-90% of these are of the clear-cell histology (NCCN GUIDELINES®, Version 3.2014 - Kidney Cancer). RCC accounts for about 3% of all cancers in the United States and an estimated 63,920 patients will be diagnosed with renal cancer and 13,860 will die of the disease in the U.S. in 2014 (Siegel *et al.,* 2014). Metastatic disease is found in about 30% of subjects at diagnosis.

For clinically localized RCC (Stage IA and IB), surgical resection, including radical nephrectomy and nephron-sparing surgery, is an effective therapy. Partial nephrectomy is generally not suitable for patients with locally advanced tumors (Stage II and III), in which case radical nephrectomy is preferred. Where the tumor is confined to the renal parenchyma, the 5-year survival rate is 60-70%, but this is lowered considerably in Stage IV disease where metastases have spread. Stage IV RCC is relatively resistant to RT and chemotherapy, although patients may benefit from surgery, and cytoreductive nephrectomy before systemic therapy is recommended for patients with a potentially surgically resectable primary and multiple resectable metastases.

Until recently, the cytokines IL-2 and IFNα, *e.g*., IFN-2b and PegIFN-2b, were the only active systemic treatments for advanced or metastatic RCC (mRCC), both providing reported objective response rates (ORRs) of 5-27%. IL-2 was approved based on a 15% durable response rate. However, due to substantial toxicities including hypotension (71%), diarrhea (67%), dyspnea (43%), rash (42%), supraventricular tachycardia (12%), and a variety of metabolic and hematologic disturbances (Fyfe *et al.,* 1995), IL-2 is only administered to relatively young and exceptionally medically fit subjects. In addition, due to the severe acute toxicities, its administration is limited to intensive care settings in tertiary care facilities. IFN-α was not approved for the treatment of RCC, and due to the limited clinical benefit and substantial toxicity profile of IL-2 and IFN-α, newer targeted agents have largely replaced cytokines in the treatment of advanced or metastatic renal cell carcinoma.

The recognition of the importance of hypoxia inducible factor alpha (HIFα) signaling in the pathogenesis of clear-cell RCC has led to widespread study of two classes of targeted therapies, anti-angiogenic TKIs and mTOR inhibitors, in 1L and 2L treatments (Mulders, 2009). Targeting of angiogenesis is rational because constitutive HIFα activation leads to the upregulation or activation of several proteins including vascular endothelial growth factor (VEGF), which can subsequently lead to tumor proliferation and neovasculature formation. Moreover, blockade of VEGF activity may modulate the immune environment and stimulate an antitumor response. Targeting of the mTOR pathway is important because activation of the upstream PI3K/Akt/mTOR signaling pathway is one method by which constitutive HIFα activation or upregulation occurs (Mulders, 2009).

Agents that target angiogenesis include VEGF-receptor (VEGFr) TKIs (*e.g*., sorafenib, sunitinib, pazopanib, axitinib, and tivozanib) and VEGF-binding mAbs (*e.g*., bevacizumab), while agents that target the mTOR pathway include the mTOR inhibitors (e.g., everolimus and temsirolimus) (Mulders, 2009; NCCN GUIDELINES®, Version 3.2014 - Kidney Cancer). However, durable responses are rare as most patients develop resistance and eventually progressive disease, and OS improvement has only been shown in one phase 3 trial in poor-risk patients: temsirolimus (TORISEL®) showed a statistically significant benefit for OS in patients with advanced RCC compared to IFNα (10.9 months versus 7.3 months) (Hudes *et al,* 2007). Everolimus (AFINITOR®) has also demonstrated a 2.1-month improvement in median progression-free survival (PFS) versus placebo, but with no OS improvement (Motzer et al*.,* 2008). Among the five approved anti-angiogenic agents (sorafenib, sunitinib, pazopanib, axitinib, and bevacizumab) and two approved mTOR inhibitors (temsirolimus, everolimus), only everolimus is approved specifically for use after the failure of treatment with anti-angiogenic therapy. In the U.S., everolimus is indicated for the treatment of advanced RCC after failure of first-line treatment with sunitinib or sorafenib, whereas in the EU, everolimus is more broadly indicated for patients with advanced RCC, whose disease has progressed on or after treatment with VEGF-targeted therapy. No recommendation exists for patients progressing on mTOR inhibitors.

### Immunotherapy of Renal Cancer

A clear need exists for effective agents for patients who have progressed on multiple lines of targeted therapy, as well as for therapies that extend survival for longer periods beyond the current standard treatments. Newer approaches to immunotherapy, beyond IL-2 and IFN-α, have recently shown promise. Immunotherapy was initially tested in patients with mRCC because traditional chemotherapy and radiotherapy had proven disappointing, renal tumors were known to be capable of evoking an immune response, and RCCs are among the most common tumors to show spontaneous regression (Elhilali *et al.,* 2000; Inman *et al.,* 2013). Currently, a variety of immunotherapeutic strategies for treating RCC are in clinical development, including T cell modulation, immune priming, and innate immunity (Rosenblatt and McDermott, 2011; Inman *et al.,* 2013). Methods of reversing T cell immunosuppression using Abs that target the PD-1 receptor or its PD-L1 ligand have also proven to be very promising in treating RCC (Topalian *et al.,* 2012a; Brahmer et al.; 2012 WO 2013/173223).

### Combination immunotherapy of renal cancer

Immunotherapeutic approaches involving a combination of agents, for example anti-PD-1 and anti-CTLA-4 Abs, have proven to be highly efficacious in treating melanoma (Wolchok et al., 2013; WO 2013/173223). By analogy, RCC patients may be able to benefit either from the combination of different immunotherapeutic drugs or the combination of such drugs with targeted agents or other treatments including, surgery, radiation, standard cancer chemotherapies, or vaccines. However, surprising and unexpected complications have sometimes been observed when immunotherapeutics are combined with other anti-cancer agents. For example, first-line therapy of two melanoma patients carrying BRAF V600E mutations with anti-PD-1 agents (nivolumab and MK-3475, respectively) did not cause significant toxicity, but treatment with vemurafenib (ZELBORAF®) upon disease progression resulted in severe hypersensitivity drug eruptions with multi-organ injury early in their vemurafenib treatment course (Johnson *et al.,* 2013). One patient subsequently developed acute inflammatory demyelinating polyneuropathy and the other developed anaphylaxis upon low-dose vemurafenib rechallenge.

Specifically relating to RCC, all agents approved to date for the treatment of mRCC have been approved as monotherapy, except for bevacizumab (AVASTIN®) which is approved only in combination with IFN-α. This combination was approved based on the improvement in PFS in conjunction with an acceptable safety profile although it does not lead to a significant improvement in median OS compared to patients receiving IFN-α alone (Escudier *et al.,* 2010; Rini *et al.,* 2010) and causes a higher incidence of Grade ≥ 3 hypertension and proteinuria. Many other combinations of anti-angiogenic therapy plus immunotherapy have been evaluated in clinical trials but significant toxicities have often been observed, including in trials of sunitinib plus IFN-α (Motzer *et al.,* 2009), sunitinib plus IL-21 (Grunwald *et al.,* 2011), sunitinib plus tremelimumab (Rini *et al.,* 2011), sorafenib plus IFN-α (Escudier *et al.,* 2007; Ryan *et al.,* 2007; Gollob *et al.,* 2007), and sorafenib plus IL-2 (Procopio *et al.,* 2011).

In a Phase 1 trial investigating the use of sunitinib plus IFN-α in treatment-naive mRCC subjects (n = 25), all subjects experienced Grade 3/4 treatment-emergent AEs, most commonly neutropenia, fatigue, and thrombocytopenia (Motzer *et al.,* 2009). Although lower starting doses were better tolerated, clinical activity was reduced. The 12% response rate seen with sunitinib plus IFN-α was lower than the historical response rate of 27.5% seen with sunitinib alone. Sunitinib plus IL-21 was also studied in a Phase 1 trial in treatment naïve subjects (n = 9) (Procopio *et al.,* 2011). The combination of full-dose sunitinib with IL-21 at 10 mcg/kg was considered too toxic based on resultant hematologic dose limiting toxicities (DLTs), while the combination with lower dose IL-21 was not considered to be therapeutically relevant. Sunitinib plus tremelimumab (an anti-CTLA-4 Ab; Ribas, 2010; U.S. Patent No. 6,682,736) was studied in a Phase 1 dose-escalation trial in 28 subjects with mRCC who had received ≤ 1 prior systemic therapy (Rini *et al.,* 2011). An unexpected toxicity of rapid-onset renal failure was observed in 4 subjects who received sunitinib 37.5 mg daily in combination with 10 mg/kg or 15 mg/kg tremelimumab every 12 weeks. Although a 43% partial response rate was observed, the toxicity of the combination at the MTD (sunitinib 37.5 mg daily plus tremelimumab 10 mg/kg q 12 weeks) was deemed unacceptable.

Combinations with sorafenib have also proven to be either too toxic or no more effective than sorafenib monotherapy. Although a Phase 1 trial of sorafenib plus IFN-α in a population including 12 mRCC subjects concluded that full dose sorafenib plus IFN 9 MIU was well tolerated with only one DLT of Grade 3 asthenia encountered (Escudier *et al.,* 2007), two subsequent Phase 2 trials found increased toxicity when this combination was used (Ryan *et al.,* 2007; Gollob *et al.,* 2007). In a Phase 2 trial of sorafenib plus IFN-α in 62 subjects with treatment-naive mRCC, the confirmed response rate for the combination (19%) was higher than the historical response rate for either agent alone, but increased toxicity, including Grade 3 or worse toxicity in 77% of subjects, limited further development (Ryan *et al.,* 2007). In a Phase 2 trial of sorafenib plus IFN-α in 40 first- or second-line subjects, the response rate was 33% and included 2 complete responses (Gollop *et al.,* 2007). However, the combination was more toxic than either drug alone, and therapy was only tolerable with treatment breaks and dose reductions (65% of subjects). Sorafenib plus subcutaneous IL-2 versus sorafenib alone was studied in a randomized Phase 2 trial in 128 subjects with treatment-naive mRCC (Procopio *et al.,* 2011). The IL-2 dose was reduced from 4.5 MIU to 3 MIU after 40 subjects were treated due to toxicity. Although the response rate was higher in the combination arm than in the sorafenib monotherapy arm (27% versus 15%, respectively), median PFS was not significantly different between the two treatment groups. The authors concluded that efficacy was not improved by use of the combination of sorafenib plus IL-2.

Thus, no combination regimen including an anti-angiogenic TKI has been able to demonstrate improved efficacy in RCC without increased, and generally unacceptable, toxicity when compared to monotherapy with either drug. The above examples in melanoma and RCC clearly indicate that combination of immunotherapy (including an immune checkpoint inhibitor drug such as an anti-CTLA-4 or anti-PD-1 Ab) with a targeted anti-cancer therapy (such as an anti-angiogenic TKI) is unpredictable and must be carefully assessed for safety as well as efficacy in clinical trials. Although the combination of nivolumab (anti-PD-1) and ipilimumab (anti-CTLA-4) has been highly effective in treating melanoma with manageable toxicity (Wolchok *et al.,* 2013), it was not hitherto known whether this combination would be significantly more effective in human subjects than treatment of RCC and other cancers with the individual agents.

### Pharmaceutical Compositions and Dosages

Therapeutic agents of the present invention may be constituted in a composition, *e.g.,* a pharmaceutical composition containing an Ab or a TKI and a pharmaceutically acceptable carrier. As used herein, a "pharmaceutically acceptable carrier" includes any and all solvents, dispersion media, coatings, antibacterial and antifungal agents, isotonic and absorption delaying agents, and the like that are physiologically compatible. Preferably, the carrier for a composition containing an Ab is suitable for intravenous, intramuscular, subcutaneous, parenteral, spinal or epidermal administration (*e.g*., by injection or infusion), whereas the carrier for a composition containing a TKI is suitable for non-parenteral, *e.g*., oral, administration. A pharmaceutical composition of the invention may include one or more pharmaceutically acceptable salts, anti-oxidant, aqueous and non-aqueous carriers, and/or adjuvants such as preservatives, wetting agents, emulsifying agents and dispersing agents.

Dosage regimens are adjusted to provide the optimum desired response, *e.g*., a maximal therapeutic response and/or minimal adverse effects. For administration of an anti-PD-1 Ab, including in combination with another anti-cancer agent, the dosage may range from about 0.01 to about 20 mg/kg, from about 0.1 to about 10 mg/kg, of the subject's body weight. For example, dosages can be about 0.1, about 0.3, about 1, about 2, about 3, about 5 or about 10 mg/kg body weight, and about 0.3, about 1, about 2, about 3, or about 5 mg/kg body weight. The dosing schedule is typically designed to achieve exposures that result in sustained receptor occupancy (RO) based on typical pharmacokinetic properties of an Ab. An exemplary treatment regime entails administration once per week, once about every 2 weeks, once about every 3 weeks, once about every 4 weeks, once about a month, once about every 3-6 months or longer. In certain embodiments, an anti-PD-1 Ab such as nivolumab is administered to the subject once about every 2 weeks. In other embodiments, the Ab is administered once about every 3 weeks. The dosage and scheduling may change during a course of treatment. For example, a dosing schedule for anti-PD-1 monotherapy may comprise administering the Ab: (i) every 2 weeks in 6-week cycles; (ii) every 4 weeks for six dosages, then every three months; (iii) every 3 weeks; (iv) 3-10 mg/kg once followed by 1 mg/kg every 2-3 weeks. Considering that an IgG4 Ab typically has a half-life of 2-3 weeks, a dosage regimen for an anti-PD-1 Ab of the invention comprises about 0.3- about 10 mg/kg body weight, preferably about 1- about 5 mg/kg body weight, more preferably about 1- about 3 mg/kg body weight via intravenous administration, with the Ab being given every 14-21 days in up to 6-week or 12-week cycles until complete response or confirmed progressive disease.

When used in combinations with other cancer agents, the dosage of an anti-PD-1 Ab may be lowered compared to the monotherapy dose. For example, a dosage of nivolumab that is significantly lower than the typical about 3 mg/kg every 3 weeks, for instance about 0.1 mg/kg or less about every 3 or 4 weeks, is regarded as a subtherapeutic dosage. In some embodiments, the subtherapeutic dose is about 2 mg/kg, about 1 mg/kg, about 0.1 mg/kg, or about 0.01 mg/kg. Receptor-occupancy data from 15 subjects who received 0.3 mg/kg to 10 mg/kg dosing with nivolumab indicate that PD-1 occupancy appears to be dose-independent in this dose range. Across all doses, the mean occupancy rate was 85% (range, 70% to 97%), with a mean plateau occupancy of 72% (range, 59% to 81%) (Brahmer *et al.,* 2010). Thus, about 0.3 mg/kg dosing may allow for sufficient exposure to lead to maximal biologic activity.

Although higher nivolumab monotherapy dosing up to 10 mg/kg every two weeks has been achieved without reaching the maximum tolerated does (MTD), the significant toxicities reported in other trials of checkpoint inhibitors plus anti-angiogenic therapy *(see, e.g.,* Johnson *et al.,* 2013; Rini *et al.,* 2011) support the selection of a nivolumab dose lower than 10 mg/kg.

Ipilimumab (YERVOY®) is approved for the treatment of melanoma at 3 mg/kg given intravenously once every 3 weeks for 4 doses. Thus, in embodiments, about 3 mg/kg is the highest dosage of ipilimumab used in combination with the anti-PD-1 Ab though, in certain embodiments, an anti-CTLA-4 Ab such as ipilimumab may be dosed within the range of about 0.3-10 mg/kg body weight about every two or three weeks when combined with nivolumab. A dosage of ipilimumab that is significantly lower than the approved 3 mg/kg every 3 weeks, for instance about 0.3 mg/kg or less about every 3 or 4 weeks, is regarded as a subtherapeutic dosage. In some embodiments, the subtherapeutic dose is about 2 mg/kg, about 1 mg/kg, about 0.1 mg/kg, or about 0.01 mg/kg.

It has been shown that combination dosing of nivolumab at 3 mg/kg and ipilimumab at 3 mg/kg exceeded the maximum tolerated dose (MTD) in a melanoma population, whereas a combination of nivolumab at 1 mg/kg plus ipilimumab at 3 mg/kg or nivolumab at 3 mg/kg plus ipilimumab at 1 mg/kg was found to be tolerable in melanoma patients (Wolchok *et al.,* 2013). Accordingly, although nivolumab is tolerated up to 10 mg/kg given intravenously every 2 weeks, in embodiments doses of the anti-PD-1 Ab do not exceed about 3 mg/kg when combined with ipilimumab. In certain embodiments, based on risk-benefit and PK-PD assessments, the dosage used comprises a combination of nivolumab at about 1 mg/kg plus ipilimumab at about 3 mg/kg, nivolumab at about 3 mg/kg plus ipilimumab at about 1 mg/kg, or nivolumab at about 3 mg/kg plus ipilimumab at about 3 mg/kg is used, each administered at a dosing frequency of once about every 2-4 weeks, once about every 3 weeks. In certain other embodiments, nivolumab is administered at a dosage of about 0.1, about 0.3, about 1, about 2, about 3 or about 5 mg/kg in combination with ipilimumab administered at a dosage of about 0.1, about 0.3, about 1, about 2, about 3 or about 5 mg/kg, once every 2 weeks, once every 3 weeks, or once every 4 weeks. In certain embodiments, the combination of an anti-PD-1 Ab and an anti-CTLA-4 Ab is administered intravenously to the subject in an induction phase about every 2 or 3 weeks for 2, 3 or 4 administrations. In certain embodiments, the combination of nivolumab and ipilimumab is administered intravenously in the induction phase about every 3 weeks for 4 administrations. The induction phase is followed by a maintenance phase during which only the anti-PD-1 Ab is administered to the subject at a dosage of about 0.1, about 0.3, about 1, about 2, about 3, about 5 or about 10 mg/kg every two or three weeks for as long as the treatment proves efficacious or until unmanageable toxicity or disease progression occurs. In certain embodiments, nivolumab is administered during the maintenance phase at a dose of about 3 mg/kg body about every 2 weeks.

For combination of nivolumab with other anti-cancer agents, in some embodiments, these agents can be administered at their approved dosages. Treatment is continued as long as clinical benefit is observed or until unacceptable toxicity or disease progression occurs. Nevertheless, in certain embodiments, the dosages of these anti-cancer agents administered are significantly lower than the approved dosage, *i.e.,* a subtherapeutic dosage, of the agent is administered in combination with the anti-PD-1 Ab. The anti-PD-1 Ab can be administered at the dosage that has been shown to produce the highest efficacy as monotherapy in clinical trials, *e.g*., about 3 mg/kg of nivolumab administered about once every three weeks, or at a significantly lower dose, *i.e.,* at a subtherapeutic dose.

The dosing schedule for a TKI varies for different TKIs. For example, the usual dosage of sunitinib comprises administration to adults of a fixed dose of 50 mg orally once a day with or without food, with dose adjustments in about 12.5-mg increments or decrements recommended based on individual patient safety and tolerability. Sunitinib is given on a schedule of four weeks on treatment followed by two weeks off treatment. In some embodiments, sunitinib is administered at a dose of about 12.5, about 25, about 37.5, about 50, about 62.5, about 75, about 87.5 or about 90 mg once a day. Sorafenib is administered to adult patients at a dosage of 400 mg orally twice a day at least one hour before or two hours after eating. In some embodiments, sorafenib is administered at a dose of about 100, about 200, about 300, about 400, about 500, about 600 or about 800 mg twice a day. Pazopanib is administered to patients at a dose of 800 mg orally once daily without food (at least 1 hour before or 2 hours after a meal). In some embodiments, pazopanib is administered at a dose of about 200, about 400, about 600, about 700, about 800, about 900, or about 1000 mg one a day. Axitinib (INLYTA®) is administered to patients at a dose of about 5 mg orally twice daily approximately 12 h apart with or without food, though dose adjustments may be made, as necessary, to about 2, about 3, about 7 or about 10 mg orally twice daily. Tivozanib is not yet approved by the FDA, but is recommended oral daily dosage at about 1 or 1.5 mg, preferably 1.5 mg, 28 days on followed by 14 days off (Eskens *et al.,* 2011) or 21 days on followed by 7 days off (Nosov *et al.,* 2012). In some embodiments, tivozanib is administered at a dose of about 0.5, about 0.75, about 1, about 1.25, about 1.5, about 1.75 or about 2 mg once a day. For a combination treatment of TKIs with an anti-PD-1 Ab, in some embodiments, the TKIs are administered at their approved or recommended dosages. Treatment is continued as long as clinical benefit is observed or until unacceptable toxicity or disease progression occurs.

In certain embodiments, the dosages of the TKI administered are the approved or recommended dosages. In other embodiments, a significantly lower dosage than the approved dosage, *i.e*., a subtherapeutic dosage, of the TKI is administered in combination with the anti-PD-1 Ab. The anti-PD-1 Ab may be administered at the dosage that has been shown to produce the highest efficacy as monotherapy in clinical trials, *e.g*., about 3 mg/kg of nivolumab administered once every three weeks (Topalian *et al.,* 2012a; Topalian *et al.,* 2012), or at a lower dose, *i.e.,* at a subtherapeutic dose. In some embodiments, the anti-PD-1 Ab is administered at a dosage of about 0.1, about 0.3, about 1, about 2, about 3 or about 5 mg/kg and the TKI is administered at a dosage of about 0.1 mg, about 0.5, about 1, about 2, about 3, about 5, about 7.5, about 10, about 12.5, about 15, about 20, about 30, about 40, about 50 or about 75 mgs/kg.

Dosage and frequency vary depending on the half-life of the Ab in the subject. In general, human Abs show the longest half-life, followed by humanized Abs, chimeric Abs, and nonhuman Abs. The dosage and frequency of administration can vary depending on whether the treatment is prophylactic or therapeutic. In prophylactic applications, a relatively low dosage is typically administered at relatively infrequent intervals over a long period of time. Some patients continue to receive treatment for the rest of their lives. In therapeutic applications, a relatively high dosage at relatively short intervals is sometimes required until progression of the disease is reduced or terminated, and preferably until the patient shows partial or complete amelioration of symptoms of disease. Thereafter, the patient can be administered a prophylactic regime.

Actual dosage levels of the active ingredients in the pharmaceutical compositions of the present invention may be varied so as to obtain an amount of the active ingredient which is effective to achieve the desired therapeutic response for a particular patient, composition, and mode of administration, without being unduly toxic to the patient. The selected dosage level will depend upon a variety of pharmacokinetic factors including the activity of the particular compositions of the present invention employed, the route of administration, the time of administration, the rate of excretion of the particular compound being employed, the duration of the treatment, other drugs, compounds and/or materials used in combination with the particular compositions employed, the age, sex, weight, condition, general health and prior medical history of the patient being treated, and like factors well known in the medical arts. A composition of the present invention can be administered via one or more routes of administration using one or more of a variety of methods well known in the art. As will be appreciated by the skilled artisan, the route and/or mode of administration will vary depending upon the desired results.

### Methods of the Invention

This disclosure provides a method of treating a subject afflicted with a renal cancer. In certain embodiments, the invention includes a method of treating a renal cancer or a subject afflicted with renal cancer comprising a combination therapy. In some embodiments, the method comprises administering to the subject a combination of therapeutically effective amounts of: (a) an anti-cancer agent which is an Ab or an antigen-binding portion thereof that specifically binds to and a PD-1 receptor and inhibits PD-1 activity; and (b) another anti-cancer therapy.. In one embodiment, the present invention is directed to a method of treating a renal cancer in a subject in need thereof or a subject afflicted with a renal cancer, which method comprises administering to the subject a combination of therapeutically effective amounts of: (a) an anti-PD-1 antibody or antigen-binding portion thereof; and (b) another anti-cancer agent. In another embodiment, the present invention is directed to a method of treating a renal cancer or a subject afflicted with a renal cancer, which method comprises administering to the subject a combination of therapeutically effective amounts of: (a) an anti-PD-1 antibody or antigen-binding portion thereof; and (b) a standard of care therapy disclosed elsewhere herein. As RCC comprises approximately 90% of renal tumors, in some embodiments the renal cancer is RCC. In other some embodiments, the subject is a human patient.

In certain embodiments, the present invention is directed to a method of treating a renal cancer in a subject in need thereof or a subject afflicted with a renal cancer, which method comprises administering to the subject an anti-PD-1 antibody or antigen-binding portion thereof, wherein the subject is concurrently treated with another anti-cancer agent. In another embodiment, the present invention is directed to a method of treating a renal cancer in a subject in need thereof or a subject afflicted with a renal cancer, which method comprises administering to the subject an anti-PD-1 antibody or antigen-binding portion thereof, wherein the subject is concurrently treated with a standard of care therapy disclosed elsewhere herein. As RCC comprises approximately 90% of renal tumors, in some embodiments the renal cancer is RCC. In other some embodiments, the subject is a human patient.

In some embodiments, the PD-1 antibody or an antigen-binding portion thereof and/or the anti-CTLA-4 antibody or an antigen-binding portion thereof are administered in combination with one or more additional anti-cancer agents selected from a group consisting of VEGF-receptor (VEGFr) TKIs (*e.g*., sorafenib, sunitinib, pazopanib, axitinib, and tivozanib), VEGF-binding mAbs and inhibitors (*e.g*., bevacizumab, aflibercept, and ziv-aflibercept), mTOR inhibitors (*e.g.*, everolimus and temsirolimus), cytokines (*e.g*., IFN-α, IFN-b2, Pegylated IFNb2 (PegIFN-b2), and IL-2), and mitotic inhibitors (*e.g*., paclitaxel, docetaxel, vincristine, eribulin, estramustine, etoposide, ixabepilone, cabazitaxel, vincristine liposome, vinorelbine, vincristine, vinblastine, and teniposide). In some embodiments, the anti-cancer agent is any other agent described herein. In certain embodiments, the therapy of the present invention (*e.g*., administration of an anti-PD-1 antibody or the administration of an anti-PD-1 antibody and another anti-cancer therapy) effectively increases the duration of survival of the subject. For example, the duration of survival of the subject is increased by at least about 1 month, at least about 2 months, at least about 3 months, at least about 4 months, at least about 5 months, at least about 6 months, at least about 7 months, at least about 8 months, at least about 9 months, at least about 10 months, at least about 11 months or at least about 1 year or more when compared to another subject treated with only either another therapy (*e.g.,* Bevacizumab or Temozolomide) or, in the case of combination therapy, only one of the two members of the combination therapy alone (*e.g*., an anti-PD-1 antibody alone). In some embodiments, the duration of survival is increased by at least about 2 months. In certain embodiments, the therapy of the present invention effectively increases the duration of progression-free survival of the subject. For example, the progression free survival of the subject is increased by at least about 1 month, at least about 2 months, at least about 3 months, at least about 4 months, at least about 5 months, at least about 6 months, at least about 7 months, at least about 8 months, at least about 9 months, at least about 10 months, at least about 11 months or at least about 1 year when compared to an untreated subject or a subject treated with only either another therapy (*e.g*., Bevacizumab or Temozolomide) or, in the case of combination therapy, only one of the two members of the combination therapy alone (*e.g*., an anti-PD-1 antibody alone). In some embodiments, the progression-free survival is increased by at least about 2 months. In certain embodiments, the therapy of the present invention effectively increases the response rate in a group of subjects. For example, the response rate in a group of subjects is increased by at least about 2%, at least about 3%, at least about 4%, at least about 5%, at least about 10%, at least about 15%, at least about 20%, at least about 25%, at least about 30%, at last about 35%, at least about 40%, at least about 45%, at least about 50%, at least about 55%, at least about 60%, at least about 70%, at least about 75%, at least about 80%, at least about 85%, at least about 90%, at least about 95%, at least about 99% or at least about 100% when compared to another group of subjects treated with only either another therapy (*e.g*., Bevacizumab or Temozolomide) or, in the case of combination therapy, only one of the two members of the combination therapy alone (*e.g.,* an anti-PD-1 antibody alone), i.e., monotherapy.

### Anti-PD-1 and anti-PD-L1 antibodies suitable for use in the disclosed methods

Anti-PD-1 Abs suitable for use in the disclosed methods are Abs that bind to PD-1 with high specificity and affinity, block the binding of PD-L1 and or PD-L2, and inhibit the immunosuppressive effect of the PD-1 signaling pathway. In any of the therapeutic methods disclosed herein, an anti-PD-1 or anti-CTLA-4 "antibody" includes an antigen-binding portion or fragment that binds to the PD-1 or CTLA-4 receptor, respectively, and exhibits the functional properties similar to those of whole Abs in inhibiting ligand binding and upregulating the immune system. In certain embodiments, the anti-PD-1 Ab or antigen-binding portion thereof cross-competes with nivolumab for binding to human PD-1. In other embodiments, the anti-PD-1 Ab or antigen-binding portion thereof is a chimeric, humanized or human monoclonal Ab or a portion thereof. In certain embodiments for treating a human subject, the Ab is a humanized Ab. In other embodiments for treating a human subject, the Ab is a human Ab. Abs of an IgG1, IgG2, IgG3 or IgG4 isotype may be used.

In certain embodiments, the anti-PD-1 Ab or antigen-binding portion thereof comprises a heavy chain constant region which is of a human IgG1 or IgG4 isotype. In certain other embodiments, the sequence of the IgG4 heavy chain constant region of the anti-PD-1 Ab or antigen-binding portion thereof contains an S228P mutation which replaces a serine residue in the hinge region with the proline residue normally found at the corresponding position in IgG1 isotype antibodies. This mutation, which is present in nivolumab, prevents Fab arm exchange with endogenous IgG4 antibodies, while retaining the low affinity for activating Fc receptors associated with wild-type IgG4 antibodies (Wang *et al.,* 2014). In yet other embodiments, the Ab comprises a light chain constant region which is a human kappa or lambda constant region. In other embodiments, the anti-PD-1 Ab or antigen-binding portion thereof is a mAb or an antigen-binding portion thereof. In certain embodiments of any of the therapeutic methods described herein comprising administration of an anti-PD-1 Ab, the anti-PD-1 Ab is nivolumab. In other embodiments, the anti-PD-1 Ab is pembrolizumab. In other embodiments, the anti-PD-1 Ab is chosen from the human antibodies 17D8, 2D3, 4H1, 4A11, 7D3 and 5F4 described in U.S. Patent No. 8,008,449. In still other embodiments, the anti-PD-1 Ab is MEDI0608 (formerly AMP-514), AMP-224, or Pidilizumab (CT-011).

In other embodiments, the invention includes a method of treating a renal cancer in a subject in need thereof or a subject afflicted with renal cancer comprising administering an anti-PD-1 antagonist or an anti-PD-1 antagonist in combination with one or more anti-cancer agents described herein to treat cancer. In some embodiments, the renal cancer is a RCC. An "anti-PD-1 antagonist" as referred herein includes any molecule that inhibits interaction between PD-1 (receptor) and PD-L1 (ligand) such that the signal pathway of PD-1/PD-L1 is blocked. PD-L1 and PD-L2 are two ligands for PD-1 that has been identified. In one embodiment, an anti-PD-1 antagonist is an anti-PD-L1 antibody. PD-L1 and PD-L2 have been shown to down-regulate T cell activation upon binding to PD-1. Both PD-L1 and PD-L2 are B7 homologs that bind to PD-1, but do not bind to other CD28 family members. PD-L1 has been shown to be abundant in a variety of human cancers. The interaction between PD-1 and PD-L1 results in a decrease in tumor infiltrating lymphocytes, a decrease in T-cell receptor mediated proliferation, and immune evasion by the cancerous cells. Immune suppression can be reversed by inhibiting the local interaction of PD-1 with PD-L1, and the effect is additive when the interaction of PD-1 with PD-L2 is blocked as well. In one embodiment, an anti-PD-Ll Ab may be substituted for the anti-PD-1 Ab in any of the therapeutic methods disclosed herein. In certain embodiments, the anti-PD-Ll Ab is BMS-936559 (formerly 12A4 or MDX-1105) (*see, e.g.,* U.S. Patent No. 7,943,743; WO 2013/173223). In other embodiments, the anti-PD-Ll Ab is MPDL3280A (also known as RG7446) (*see, e.g.,* Herbst et al. 2013; U.S. Patent No. 8,217,149) or MEDI4736 (Khleif, 2013).

In another embodiment, an anti-PD-1 antagonist is a soluble PD-1 protein. In other embodiments, an anti-PD-1 antagonist is a PD-1-Fc fusion protein. In some embodiments, the PD-1-Fc fusion protein is the result of the fusion of any PD-1 antibody described herein with an Ig Fc domain. In some embodiments, the Ig Fc domain is an IgG, IgA or an IgM domain. In some embodiments, fusion proteins may be created by chemical synthesis or by creation of a polynucleotide that encodes the desired fusion protein.

In certain embodiments, an anti-PD-1 antagonist includes an anti-PD-1 fusion protein, an antisense molecule, a small molecule, a ribozyme, or a nanobody that inhibits or prevents interaction between PD-1 and PD-L1.

### Combination of an anti-PD-1 Ab with an anti-angiogenic TKI for treating RCC

In certain embodiments of the disclosed methods for treating RCC, the other anti-cancer agent, *i.e.,* the anti-cancer agent other that the anti-PD-1 Ab which is used in combination with the anti-PD-1 Ab, is an anti-angiogenic TKI. Various TKIs that have been approved for treating RCC or have demonstrated efficacy in clinical trials, including sorafenib, sunitinib, pazopanib, axitinib, and tivozanib, may be used in the disclosed combination therapy methods with an anti-PD-1 Ab. However, until the efficacy and safety of these combinations are assessed in pre-clinical or in clinical trials, there is the possibility that unexpected toxicities or lack of significantly enhanced efficacy relative to the monotherapies may result. The preclinical data disclosed herein in the Renca murine RCC model indicate that an anti-PD-1 Ab interacts synergistically with sunitinib to inhibit RCC tumor growth whereas the combination of an anti-PD-1 Ab and sorafenib did not significantly enhance the low level of anti-tumor activity of sorafenib in this murine RCC model (Figures 1 and 2). Further to the demonstration that the combination of an anti-PD-1 Ab and sunitinib is highly effective in treating RCC in the murine model, the efficacy and safety of this combination, as well as the combination of nivolumab and pazopanib, in human RCC patients has been verified in clinical studies.

Example 5 provides interim results of an ongoing Phase 1 trial (NCT01472081; *see* Example 4) of nivolumab in combination with sunitinib or pazopanib in patients with mRCC. The data show that nivolumab in combination with either of these TKIs exhibit significantly higher antitumor activity in these patients and a manageable safety profile (*see* Figure 12). Accordingly, in certain embodiments of the present combination therapy methods, the anti-angiogenic TKI is sunitinib. In other embodiments, the TKI is pazopanib. In further embodiments, the TKI is axitinib. Axitinib is a highly selective, potent inhibitor of VEGF receptors 1, 2, and 3 that has a favorable toxicity profile (Sonpavde *et al.,* 2008) and been approved by the FDA for the treatment of advanced RCC after the failure of one prior systemic therapy. In other embodiments, the TKI is sorafenib. In yet further embodiments, the TKI is tivozanib. Tivozanib is a potent, selective, long half-life inhibitor of VEGFR-1, -2, and -3 that is undergoing clinical trials for the treatment of RCC. Axitinib and tivozanib have not been tested in either preclinical or clinical studies herein. However, based on their highly selective inhibition of all three VEGF receptors, which overlaps with the VEGFR inhibitory activity of sunitinib, pazopanib and sorafenib while minimizing off-target toxicities, both axitinib and tivozanib are excellent candidates for combining with an anti-PD-1 Ab in the disclosed therapeutic methods.

In certain embodiments, administration of the anti-PD-1 Ab or antigen-binding portion thereof and anti-angiongenic TKI results in an increase in the infiltration of the cancer tissue by T cells compared to an untreated subject or a subject treated with a monotherapy, either of the anti-PD-1 antibody or the other anti-cancer agent. As described in Example 2, combination therapy can lead to an increase in the localization of, *e.g.,* CD4⁺ and CD8⁺ T cells into a renal tumor. This increase in T cell infiltration can be at least about 10%, at least about 20%, at least about 30%, at least about 40%, at least about 50%, at least about 60%, at least about 70%, at least about 80%, or at least about 90% greater than the level of T cell infiltration in an untreated subject or a subject treated with a monotherapy, either of the anti-PD-1 antibody or the other anti-cancer agent. The disclosed methods can further result in increased T cell proliferation compared to an untreated subject or a subject treated with a monotherapy, either of the anti-PD-1 antibody or the other anti-cancer agent. T cell proliferation can be increased by at least about 10%, at least about 20%, at least about 30%, at least about 40%, at least about 50%, at least about 60%, at least about 70%, at least about 80%, or at least about 90%. The disclosed methods can also, in certain embodiments, lead to a decrease in T-regulatory cells compared to an untreated subject or a subject treated with a monotherapy, either of the anti-PD-1 antibody or the other anti-cancer agent.

In other embodiments, the disclosed methods for treating RCC decreases the number of monocytic myeloid-derived suppressor cells or increases the number of granulocytic myeloid cells, compared to an untreated subject or a subject treated with a monotherapy, either of the anti-PD-1 antibody or the other anti-cancer agent. In an embodiment, the monocytic myeloid-derived suppressor cells are characterized by CD11b⁺/Ly6C^{hi}/Ly6G⁻ expression or CD11b⁺/Ly6C^{low}/Ly6G⁻ expression. In another embodiment, the granulocytic myeloid cells are characterized by CD11b⁺/Ly6C⁻/Ly6G⁺ expression. The number of monocytic myeloid-derived suppressor cells can decrease by at least about 5%, at least about 10%, at least about 15%, at least about 20%, at least about 25%, at least about 30%, at least about 40%, at least about 50%, at least about 60%, at least about 70%, at least about 80%, or at least about 90%. The number of granulocytic myeloid cells can increase by at least about 5%, at least about 10%, at least about 20%, at least about 30%, at least about 40%, at least about 50%, at least about 60%, at least about 70%, at least about 80%, or at least about 90%.

### Dosages of anti-PD-1 Abs and anti-angiogenic TKIs used in combination therapy

In certain embodiments of the present combination therapy methods, the therapeutically effective dosage of the anti-PD-1 Ab or antigen-binding portion thereof comprises a dose ranging from about 0.1 to about 10.0 mg/kg body weight which is administered by intravenous infusion about once per week, about once every 2 weeks, about once every 3 weeks, or about once a month, in combination with an anti-angiogenic TKI. In certain embodiments, the anti-PD-1 Ab is administered at a dose of 2 mg/kg once every 3 weeks. In other embodiments, the anti-PD-1 Ab is administered at a dose of about 5 mg/kg once about every 3 weeks.

Dosages of sunitinib may comprise, for example, administration to an adult RCC patient of a dose ranging from about 25 to about 50 mg, in some embodiments 50 mg, in a 42-day cycle comprising daily administration for 28 days and no administration for 14 days, and repetition of the cycle for as long as clinical benefit is observed or until unmanageable toxicity or disease progression occurs. Alternatively, administration of the dose of 25 to 50 mg administration for 28 days and no administration for 14 days may be followed by daily administration of sunitinib at 37.5 mg for as long as clinical benefit is observed or until unmanageable toxicity or disease progression occurs. In some embodiments, sunitinib is administered at a dose of about 5, about 10, about 15, about 20, about 25, about 30, about 35, about 40, about 45, about 50, about 55, about 60, about 65, about 70 or about 75 mg/kg body weight.

Pazopanib is administered at a dosage of about 50, about 100, about 200, about 300, about 400, about 500, about 600, about 700 or about 800 mg orally once daily in combination with the anti-PD-1 Ab. In some embodiments, pazopanib is administered at its recommended dose of 800 mg once daily without food.

Dosages of TKIs other than sunitinib and pazopanib that have not been tested in the clinical trial in combination with the anti-PD-1 Ab are based on known therapeutic dosages. Thus, in some embodiments, sorafenib is administered at its recommended dosage of 400 mg orally twice a day. In some embodiments, axitinib (INLYTA®) is administered to patients at its recommended dose of 5 mg orally twice daily approximately 12 h apart. In some embodiments, tivozanib is administered at its recommended oral daily dose of 1.5 mg, 28 days on followed by 14 days off (Eskens *et al.,* 2011) or 21 days on followed by 7 days off (Nosov *et al.,* 2012).

In embodiments, nivolumab is administered at about 2 or about 5 mg/kg by intravenous infusion on Days 1 and 22 of each 6-week (42-day) cycle in combination with the TKI, *e.g*., sunitinib about 50 mg orally administered on Days 1 - 28 of each 42 day cycle, or pazopanib about 800 mg orally administered on Days 1 - 42 of each 42 day cycle. Other TKIs employed in the present methods are administered according to their approved or recommended dosage regimens. Treatment is continued as long as clinical benefit is observed or until unacceptable toxicity or disease progression occurs.

The synergistic interaction observed in preclinical models between, for example, anti-PD-1 and sunitinib suggests that one or both of these therapeutics may be administered to a patient at subtherapeutic dosages, *i.e.,* a dose of the therapeutic agent that is significantly lower than the usual or FDA-approved dose when administered as monotherapy for the treatment of the cancer. In certain embodiments of the disclosed invention, the anti-PD-1 Ab or antigen-binding portion thereof is administered to a RCC patient at a subtherapeutic dose. In other embodiments, the TKI is administered at a subtherapeutic dose described herein. In further embodiments, the anti-PD-1 Ab or antigen-binding portion thereof and the TKI are each administered at a subtherapeutic dose described herein.

The combination therapy of the anti-PD-1 antibody or antigen-binding portion thereof and the other anti-cancer agent can be administered concurrently or in sequence (*e.g.,* the anti-PD-1 antibody or antigen-binding portion thereof administered first and the other anti-cancer agent administered second or the other anti-cancer agent administered first and the anti-PD-1 antibody or antigen-binding portion thereof administered second).

### Combination of an anti-PD-1 Ab with an anti-CTLA-4 Ab for treating RCC

This disclosure also provides combination therapy methods for treating RCC wherein an anti-PD-1 Ab is combined with another anti-cancer agent which is an Ab or an antigen-binding portion thereof that binds specifically to CTLA-4 and inhibits CTLA-4 activity. It has been demonstrated herein (*see* Example 6) that the combination of the anti-PD-1 Ab, nivolumab, and the anti-CTLA-4 Ab, ipilimumab, produces a level of clinical activity, as measured by ORR, that is significantly higher than the activity observed with nivolumab or ipilimumab alone. Accordingly, in certain embodiments, the anti-CTLA-4 Ab that is used in combination with the anti-PD-1 Ab is ipilimumab. In embodiments, the anti-CTLA-4 Ab is tremelimumab. In other embodiments, the anti-CTLA-4 Ab or antigen-binding portion thereof is an Ab or portion thereof that cross-competes with ipilimumab for binding to human CTLA-4. In certain other embodiments, the anti-CTLA-4 Ab or antigen-binding portion thereof is a chimeric, humanized or human mAb or a portion thereof. In yet other embodiments, the anti-CTLA-4 Ab or antigen-binding portion thereof comprises a heavy chain constant region which is of a human IgG1 or IgG4 isotype. In embodiments, the anti-CTLA-4 Ab comprises a heavy chain constant region which is of a human IgG1 isotype.

For the combination of an anti-PD-1 and an anti-CTLA-4 Ab, the dosing regimen comprises an induction period (also referred to herein as an induction phase) during which one or more, preferably about four, combination doses of the anti-PD-1 and anti-CTLA-4 Abs are administered to the patient, followed by a maintenance period or phase comprising dosing with the anti-PD-1 Ab alone, *i.e.,* not including the anti-CTLA-4 Ab. In certain embodiments, the method comprises (a) an induction phase, wherein the anti-PD-1 and anti-CTLA-4 antibodies or antigen-binding portions thereof are administered in combination in 2, 4, 6, 8 or 10 doses, each dose ranging from about 0.1 to about 10.0 mg/kg body weight administered at least once about every 2 weeks, once about every 3 weeks, or once about every 4 weeks, followed by (b) a maintenance phase, wherein no anti-CTLA-4 antibody or antigen-binding portion thereof is administered and the anti-PD-1 antibody or antigen-binding portion thereof is repeatedly administered at a dose ranging from about 0.1 to about 10 mg/kg at least once about every 2 weeks, once about every 3 weeks, or once about every 4 weeks.

In certain embodiments, the anti-PD-1 Ab or antigen-binding portion thereof is administered at a subtherapeutic dose. In certain other embodiments, the anti-CTLA-4 Ab or antigen-binding portion thereof is administered at a subtherapeutic dose. In further embodiments, both the anti-PD-1 Ab or antigen-binding portion thereof and the anti-CTLA-4 Ab or antigen-binding portion thereof are each administered at a subtherapeutic dose. In some embodiments anti-PD-1 Ab or antigen-binding portion thereof and/or the anti-CTLA-4 Ab or antigen-binding portion thereof is administered at a therapeutic dose.

In certain embodiments, (a) the induction phase comprises at least 4 doses administered at 3-week intervals, wherein the anti-PD-1 and anti-CTLA-4 Abs are administered at the following dosages: (i) 0.1 mg/kg anti-PD-1 Ab and 3 mg/kg of anti-CTLA-4 Ab; (ii) 0.3 mg/kg anti-PD-1 Ab and 3 mg/kg of anti-CTLA-4 Ab; (iii) 1 mg/kg anti-PD-1 Ab and 3 mg/kg of anti-CTLA-4 Ab; (iv) 3 mg/kg anti-PD-1 Ab and 3 mg/kg of anti-CTLA-4 Ab; (v) 5 mg/kg anti-PD-1 Ab and 3 mg/kg of anti-CTLA-4 Ab; (vi) 10 mg/kg anti-PD-1 Ab and 3 mg/kg of anti-CTLA-4 Ab; (vii) 0.1 mg/kg anti-PD-1 Ab and 1 mg/kg of anti-CTLA-4 Ab; (viii) 0.3 mg/kg anti-PD-1 Ab and 1 mg/kg of anti-CTLA-4 Ab; (ix) 1 mg/kg anti-PD-1 Ab and 1 mg/kg of anti-CTLA-4 Ab; (x) 3 mg/kg anti-PD-1 Ab and 1 mg/kg of anti-CTLA-4 Ab; (xi) 5 mg/kg anti-PD-1 Ab and 1 mg/kg of anti-CTLA-4 Ab; or (xii) 10 mg/kg anti-PD-1 Ab and 1 mg/kg of anti-CTLA-4 Ab, and (b) the maintenance phase comprises repeated administration of the anti-PD-1 Ab at a dose of 3 mg/kg every 2 weeks.

Because of durability of the clinical effect previously demonstrated with immunotherapy by inhibition of immune checkpoints (*see, e.g.,* WO 2013/173223), the maintenance phase may include, in alternative embodiments, a finite number of doses, *e.g.,* 1-10 doses, or may involve dosing at long intervals, *e.g.,* once about every 3-6 months or once about every 1-2 years or longer intervals. The maintenance phase may be continued for as long as clinical benefit is observed or until unmanageable toxicity or disease progression occurs.

Given the uncertainty of whether the ipilimumab administered past week 12 contributes to clinical benefit in melanoma and the fact that the U.S. Food and Drug Administration (FDA)- and European Medicines Agency (EMA)-approved schedule for YERVOY® is every 3 weeks for a total of 4 doses, in embodiments the anti-CTLA-4 Ab is administered during the induction phase once every 3 weeks for a total of 4 doses. Accordingly, in certain embodiments, the method comprises (a) an induction phase consisting of 4 combination doses administered at 3-week intervals, wherein (i) the anti-PD-1 antibody or antigen-binding portion thereof is administered at 3 mg/kg body weight and the anti-CTLA-4 antibody or antigen-binding portion thereof is administered at 1 mg/kg body weight; (ii) the anti-PD-1 antibody or antigen-binding portion thereof is administered at 1 mg/kg body weight and the anti-CTLA-4 antibody or antigen-binding portion thereof is administered at 3 mg/kg body weight; or (iii) the anti-PD-1 antibody or antigen-binding portion thereof is administered at 3 mg/kg body weight and the anti-CTLA-4 antibody or antigen-binding portion thereof is administered at 3 mg/kg body weight; and (b) the maintenance phase comprises repeated administration of the anti-PD-1 antibody or antigen-binding portion thereof at a dose of 3 mg/kg every 2 weeks. In further embodiments of these methods, the maintenance phase is continued for as long as clinical benefit is observed or until unacceptable or unmanageable toxicity or disease progression occurs.

In certain embodiments of the present methods, the anti-PD-1 Ab is nivolumab. In other embodiments, it is pembrolizumab. In yet other embodiments, the anti-CTLA-4 Ab is ipilimumab. In further embodiments, the anti-CTLA-4 Ab is tremelimumab. Typically, the anti-PD-1 and anti-CTLA-4 Abs are formulated for intravenous administration. In some embodiments, the combination of the anti-PD-1 antibody or an antigen-binding portion thereof and the anti-CTLA-4 antibody or an antigen-binding portion thereof is administered concurrently as a single composition or as separate compositions. In some embodiments, the anti-PD-1 and anti-CTLA-4 Abs are administered sequentially. In some embodiments, the anti-PD-1 and anti-CTLA-4 Abs are administered sequentially during the induction phase. In certain embodiments, when the anti-PD-1 and anti-CTLA-4 Abs are administered in combination, they are administered within 30 minutes of each other. Either Ab may be administered first, that is, in certain embodiments, the anti-PD-1 Ab is administered before the anti-CTLA-4 Ab, whereas in other embodiments, the anti-CTLA-4 Ab is administered before the anti-PD-1 Ab. Typically, each Ab is administered by intravenous infusion over a period of 60 minutes. In certain embodiments, the anti-PD-1 and anti-CTLA-4 Abs are administered concurrently, either admixed as a single composition in a pharmaceutically acceptable formulation for concurrent administration, or concurrently as separate compositions with each Ab in a pharmaceutically acceptable formulation.

Certain embodiments of the present methods comprise (a) an induction phase consisting of administration of nivolumab by intravenous infusion followed by administration of ipilimumab by intravenous infusion every 3 weeks for 4 combination doses, followed by (b) maintenance dosing with nivolumab administered by intravenous infusion every 2 weeks starting 3 weeks after the 4th dose of induction therapy or after Day 113 if the 4th dose of induction therapy has not been administered due to treatment delays.

In certain embodiments, a combination of the anti-PD-1 Ab or antigen-binding portion thereof, an anti-angiogenic TKI, and an anti-CTLA-4 Ab or antigen-binding portion thereof can be administered to a subject for treating RCC. In some embodiments, the anti-angiogenic TKI is sorafenib, sunitinib, pazopanib, axitinib, or tivozanib. In certain embodiments, the anti-angiogenic TKI is sunitinib or pazopanib. In another embodiment, the anti-CTLA-4 ab is ipilimumab or tremelimumab. In one particular embodiment, the combination of the anti-PD-1 Ab or antigen-binding portion thereof, an anti-angiogenic TKI, and an anti-CTLA-4 Ab or antigen-binding portion thereof is administered at a dose selected from (i) 0.1 mg/kg anti-PD-1 Ab and 3 mg/kg of anti-CTLA-4 Ab; (ii) 0.3 mg/kg anti-PD-1 Ab and 3 mg/kg of anti-CTLA-4 Ab; (iii) 1 mg/kg anti-PD-1 Ab and 3 mg/kg of anti-CTLA-4 Ab; (iv) 3 mg/kg anti-PD-1 Ab and 3 mg/kg of anti-CTLA-4 Ab; (v) 5 mg/kg anti-PD-1 Ab and 3 mg/kg of anti-CTLA-4 Ab; (vi) 10 mg/kg anti-PD-1 Ab and 3 mg/kg of anti-CTLA-4 Ab; (vii) 0.1 mg/kg anti-PD-1 Ab and 1 mg/kg of anti-CTLA-4 Ab; (viii) 0.3 mg/kg anti-PD-1 Ab and 1 mg/kg of anti-CTLA-4 Ab; (ix) 1 mg/kg anti-PD-1 Ab and 1 mg/kg of anti-CTLA-4 Ab; (x) 3 mg/kg anti-PD-1 Ab and 1 mg/kg of anti-CTLA-4 Ab; (xi) 5 mg/kg anti-PD-1 Ab and 1 mg/kg of anti-CTLA-4 Ab; or (xii) 10 mg/kg anti-PD-1 Ab and 1 mg/kg of anti-CTLA-4 Ab, wherein the TKI is administered at about its recommended dose. For example, sunitinib can be administered at a dosage with the range of about 25 to about 50 mg, in some embodiments 50 mg; pazopanib can be administered at a dosage of about 200, about 400 or about 800 mg; sorafenib can be administered at a dosage of about 400 mg, twice daily; and tivozanib can be administered at its recommended oral daily dose of about 1.5 mg daily.

### Combination of an anti-PD-1 Ab with other known anti-cancer drugs for treating RCC

One of skill in the art would recognize that the described methods of treating RCC with the anti-PD-1 antibodies or antigenic fragments thereof can be combined with other known treatments of RCC. For example, the anti-PD-1 Ab or antigenic fragment thereof can be combined with a mitotic inhibitor, such as paclitaxel, docetaxel, vincristine, eribulin, estramustine, etoposide, ixabepilone, cabazitaxel, vincristine liposome, vinorelbine, vincristine, vinblastine, or teniposide. Various cytokines are also known to be useful in treating cancer, including, as discussed above, IFN-α or IL-2. The anti-PD-1 Ab or antigenic fragment thereof can be combined with a cytokine, such as IFN-α, IFN-b2, Pegylated IFNb2 (PegIFN-b2), or IL-2. Other small molecule treatments known in the art may also be suitable, including, but not limited to, vinflunine. In some embodiments, the anti-PD-1 antibodies described herein, including combination treatments of anti-PD-1 antibodies and TKIs or anti-CTLA-4 antibodies, are used in combination with any other treatment described herein, including SOC treatments.

The anti-PD-1 antibodies can be administered in combination with one or more of the other known anti-cancer drugs described herein at a dosing that is standard for the particular anti-cancer drug. For example, pazopanib can be administered at a dosage of about 400 to about 800 mg; Axitinib can be administered at a dosage of about 1 mg, about 2 mg, about 3 mg, about 4 mg, about 5 mg, about 6 mg, about 7 mg, about 8 mg, about 9 mg, or at least about 10 mg; paclitaxel and/or docetaxel can be administered at a dosage of about 10 mg/m², about 25 mg/m², about 30 mg/m², about 35 mg/m², about 40 mg/m², about 45 mg/m², about 50 mg/m², about 75 mg/m², about 100 mg/m², about 125 mg/m², about 150 mg/m², about 175 mg/m², about 200 mg/m², about 225 mg/m², about 250 mg/m², about 275 mg/m², or at least about 300 mg/m²; and vinflunine can be administered at a dose of about 50 mg/m², about 75 mg/m², about 100 mg/m², about 125 mg/m², about 150 mg/m², about 175 mg/m², about 200 mg/m², about 225 mg/m², about 250 mg/m², about 275 mg/m², about 300 mg/m², about 325 mg/m², about 350 mg/m², about 375 mg/m², about 400 mg/m², about 425 mg/m², about 450 mg/m², about 475 mg/m², or at least about 500 mg/m².

### Kits

Also within the scope of the present invention are kits comprising an anti-PD-1 Ab and a TKI for therapeutic uses. Kits typically include a label indicating the intended use of the contents of the kit and instructions for use. The term label includes any writing, or recorded material supplied on or with the kit, or which otherwise accompanies the kit. Accordingly, this disclosure provides a kit for treating a subject afflicted with a renal cancer, the kit comprising: (a) a dosage ranging from 0.1 to 10 mg/kg body weight of an anti-cancer agent which is an Ab or an antigen-binding portion thereof that specifically binds to the PD-1 receptor and inhibits PD-1 activity; (b) a dosage of another anti-cancer agent which is (i) an anti-angiogenic TKI, for example, sunitinib at a dosage of 12.5, 25 or 50 mg once daily on a schedule of four weeks on treatment followed by two weeks off treatment, pazopanib at a dosage of 200, 400 or 800 mg once daily, or sorafenib at a dosage of 400 mg once or twice daily; or (ii) a dosage ranging from 0.1 to 10 mg/kg body weight of an antibody or an antigen-binding portion thereof that specifically binds to and inhibits CTLA-4; and (c) instructions for using the anti-PD-1 Ab and the other anti-cancer agent in any of the combination therapy methods disclosed herein. In certain embodiments, the anti-PD-1, the anti-CTLA-4 Ab and/or the TKI may be co-packaged in unit dosage form. In certain embodiments for treating human patients, the kit comprises an anti-human PD-1 Ab disclosed herein, *e.g*., nivolumab or pembrolizumab. In other embodiments, the kit comprises an anti-human CTLA-4 Ab disclosed herein, *e.g*., ipilimumab or tremelimumab.

The present invention is further illustrated by the following examples which should not be construed as further limiting. The contents of all references cited throughout this application are expressly incorporated herein by reference.

### EXAMPLE 1

### Treatment of Renal Cancer with Anti-PD-1 and TKIs in Mouse Model

### Materials and Methods

### Animals

Female 8-12 weeks old Balb/c and C57/BL6 mice (Harlan Laboratories, Federick, MD) were used in all studies. Mice were housed in sterile microisolator cages and administered sterile food and water *ad libitum,* unless otherwise specified.

### Reagents

Sunitinib (SUTENT®) is an orally available, small-molecule, multikinase inhibitor targeting several receptor tyrosine kinases (including VEGFR-1, VEGFR-2 and VEGFR-3, PDGFR-α and -β, c-Kit, FLT-3, CSF-1R and RET) that is approved by the FDA for the treatment of renal cell carcinoma. Sunitinib has been reported to exhibit immunomodulatory effects, including a reduction in myeloid-derived suppressor cells (Ko *et al.,* 2009; Ko *et al.,* 2010) and a reduction in T-regulatory suppressor cells (Hipp *et al.,* 2008; Ozao-Choy *et al.,* 2009).

Sorafenib (NEXAVAR®) is an orally available, small-molecule multikinase inhibitor (cRAF, bRAF, KIT, FLT-3, VEGFR-2, VEGFR-3, and PDGFR-β) that was approved by the FDA for the treatment of primary kidney cancer (advanced renal cell carcinoma), advanced primary liver cancer, and radioactive iodine-resistant advanced thyroid carcinoma. Sorafenib has been reported to inhibit T-cell proliferation by inducing apoptosis (Houben *et al.,* 2009; Molhock *et al.,* 2009), reduce the induction of antigen-specific T cells and reduce dendritic cell maturation (Hipp *et al.,* 2008).

The anti-PD-1 mAb, 4H2, is a chimeric rat-mouse anti-mouse PD-1 Ab (U.S. Patent No. 8,008,449) that was purified and certified to have < 0.5 EU/mg endotoxin level, > 95% purity and < 5% high molecular-weight species.

The murine renal carcinoma (Renca) and murine colon adenocarcinoma (CT-26) tumor cell lines were maintained *in vitro* for less than 10 passages.

### Efficacy studies

Tumor cells were implanted subcutaneously (SC). When the mean tumor volume reached about 100 mm³, mice were randomized into groups of 8 animals. Vehicle (control), sunitinib (120 mg/kg) or sorafenib (200 mg/kg) was orally administered once daily for 14 days (*QDx14, PO*)*.* MAb 4H2 was administered at 10 mg/kg every 4 days for a total of 4 treatments (*Q4Dx4*) intraperitoneally (*IP*)*.* Tumors were measured twice weekly, and the tumor volume was calculated as length x (width²/2). Synergy was defined as excess over highest single agent (EOHSA), *i.e.,* a combination was considered synergistic if the antitumor effect was significantly greater than the effect observed with monotherapy (*P* < 0.05, Wilcoxon sign-rank test).

### Results

The effects of treatment with anti-PD-1 and sunitinib or sorafenib as monotherapy or combination therapy on the median volume of implanted RCC mouse tumors are shown in Figure 1. Sunitinib monotherapy showed activity in this Renca murine RCC model producing tumor growth inhibition (TGI) of 85% by the end of treatment (Day 33; *see* Table 1); however, tumors grew progressively after cessation of therapy (*see* Figure 1). When administered as monotherapy the anti-PD-1 mAb was inactive in this model, but the combination of anti-PD-1 and sunitinib produced significant antitumor activity (TGI of 101%) resulting in complete tumor regressions (Table 1) or marked delay in tumor growth up to the end of monitoring 50 days post-implantation (Figure 1). These results, which were observed in two independent studies of the same model, show a synergistic interaction between a nivolumab murine surrogate anti-PD-1 mAb and sunitinib in the murine RCC (Renca) tumor model since the effect of this combination in inhibiting tumor growth is considerably greater than the sum of the level of inhibition exhibited by each therapeutic agent individually.

In contrast, whereas sorafenib monotherapy was active in this RCC model (62% TGI; Table 1), combination treatment with the anti-PD-1 mAb and sorafenib did not significantly enhance the anti-tumor activity of sorafenib in this murine model and did not result in synergistic efficacy (Figure 1).

Figure 2 shows the time (in days) taken for the tumor to reach a volume of 500 mm³. Consistent with the data in Figure 1, the growth of tumors treated with the anti-PD-1/sunitinib combination was significantly inhibited and these tumors took about 63 days to reach 500 mm³, significantly longer than the 19-34 days taken by tumors treated with the control, anti-PD-1 Ab, sunitinib, sorafenib, or anti-PD-1/sorafenib combination.

The percentage tumor growth inhibition, measured at 33 days post-implantation, is shown in Table 1. These data are consistent with the data shown in Figures 1 and 2, confirming that tumor growth is completely inhibited by the combination of anti-PD-1 and sunitinib, whereas the other treatments cause a lower level of tumor growth inhibition. The superior efficacy exhibited by the combination of anti-PD-1 and sunitinib over the combination of anti-PD-1 and sorafenib is consistent with the different immunomodulatory effects mediated by sunitinib and sorafenib, respectively, which suggest that sunitinib, but not sorafenib, is suitable for combination with immunotherapeutic approaches for treatment of cancer patients (*see* Hipp *et al.,* 2008). However, the effects of a combination of anti-PD-1 and sorafenib in human subjects may not entirely reflect the effects observed in the mouse RCC model, and this combination may be highly effective in treating human patients.

**Table 1. Tumor growth inhibition (TGI) and objective response rate mediated by tested therapeutic agents 33 days after implantation**

| Treatment | % TGI | Objective Responses (ORs) |
|---|---|---|
| Control | - | - |
| Sunitinib (120 mg/kg *QDx14 D*) | 85 | 0/8 |
| Sorafenib (200 mg/kg *QDx14 D)* | 62 | 0/8 |
| Anti-PD-1 mAb 4H2 (10 mg/kg *Q4Dx4*) | 51 | 0/8 |
| Anti-PD-1 mAb 4H2 (10 mg/kg *Q4Dx4)* + sunitinib (120 mg/kg *QDx14 D)* | 101 | 3/8 CR; 2/8 PR |
| Anti-PD-1 mAb 4H2 (10 mg/kg *Q4Dx4)* + sorafenib (200 mg/kg *QDx14 D)* | 77 | 2/8 CR |

| | | |
|---|---|---|
| CR = complete regression; PR = partial regression | | |

The effects of the various treatments on overall body weight of the mice are shown in Figure 3. The combination of anti-PD-1 and sunitinib was well tolerated, as evidenced by a lack of decrease in body weight of the mice. The combination of anti-PD-1 and sorafenib was also well tolerated, though apparently slightly less so than the combination of anti-PD-1 and sunitinib as the sorafenib combination caused a small but noticeable reduction in body weight.

### EXAMPLE 2

### Immune Cell Infiltration of Tumors Post-Treatment with Anti-PD-1 and Sunitinib

### Materials and Methods

Immunohistochemistry and flow cytometry analyses were used to assess immune cell infiltration of tumors.

### Immunohistochemistry

Frozen sections were fixed in an acetone/methanol mix and rinsed in phosphate-buffered saline (PBS). Sections were blocked for endogenous peroxidase and the primary antibodies (anti-CD8, anti-CD4, anti-FoxP3, and anti-PD-Ll) were added and incubated overnight. Chromogenic development was done using a brown polymer-based detection system (Biocare Medical), with hematoxylin used as a blue counter stain for visualization of cellular nuclei.

### Immunophenotyping by flow cytometry

Tumor-infiltrating immune cells were isolated and prepared as a single-cell suspension, then stained and analyzed via flow cytometry for expression of T-cell subsets (regulatory and activated) and myeloid cells (*i.e.,* for expression of CD3, CD4, CD8, FoxP3, CD25, and CD69).

### Results

In control mice, immunohistochemical (IHC) analysis detected CD4⁺ and CD8⁺ T cells in the tumor periphery (Figure 4). When animals were dosed with sunitinib, there was an influx of CD4⁺ and CD8⁺ T cells predominantly to the periphery and along the vasculature. In contrast, greater infiltration of CD4⁺ and CD8⁺ T cells was observed throughout the tumor when the mice were dosed with the combination of anti-PD-1 mAb and sunitinib. The combination of anti-PD-1 mAb and sunitinib resulted in greater numbers of tumor-infiltrating CD4⁺ and CD8⁺ T cells compared with each agent alone or control vehicle (Figure 4). In contrast, increased T-cell infiltration into the tumor was not observed with anti-PD-1 mAb or sorafenib alone, or with the combination of anti-PD-1 mAb and sorafenib, versus the control group (data not shown). Sunitinib and sorafenib have been shown to exhibit immunomodulatory effects, which may be implicated in the different therapeutic effects observed when these agents are combined with the anti-PD-1 Ab immune checkpoint inhibitor.

Flow cytometry analyses of tumor-infiltrating immune cells showed marked differences within the groups treated with anti-PD-1 mAb in combination with sunitinib or sorafenib (Figures 5-7). Compared to the combination of anti-PD-1 mAb and sorafenib, the combination of anti-PD-1 mAb and sunitinib resulted in (i) a greater frequency of proliferating CD4⁺/Ki67⁺, CD8⁺, and CD8⁺/Ki67⁺(*P* < 0.05; Figures 5 and 6); (ii) reduced percentages of monocytic myeloid-derived suppressor cells, as defined by CD11b⁺/Ly6C^{hi}/Ly6G⁻ (*P* < 0.05), monocytic cells expressing CD11b⁺/Ly6C^{low}/Ly6G⁻, or both (P < 0.05; Figure 7); and (iii) an increased percentage of granulocytic myeloid cells CD11b⁺/Ly6C⁻/Ly6G⁺ (*P* < 0.05; Figure 7). Thus, treatment with the combination of sunitinib and anti-PD-1 mAb induced changes in the composition of the tumor microenvironment that may support an effective antitumor immune response. Low numbers of T-regulatory cells (CD45⁺/CD4⁺/CD25⁺/FoxP3⁺) were detected across the experimental groups, making it difficult to determine treatment effects (data not shown).

### EXAMPLE 3

### Enhancement of In Vivo Cytotoxicity of Antigen-Specific Immune Response

### Materials and Methods

### Cytotoxicity assay

The CT26 murine colon cancer model that expresses the AH1 antigen was used in experiments to determine whether treatments had any effect on antigen-specific T cells. Five days following SC implantation with CT26 cells, mice were treated with vehicle or anti-PD-1 mAb 10 mg/kg, 2 doses given 3 days apart (*Q3Dx2*)*,* IP with or without sunitinib 120 mg/kg once daily for 5 days (*QDx5*)*.* Cell killing was determined on days 2 and 4 following final treatment.

One day prior to assay, splenocytes from naive BALB/c donors were incubated with or without CT26 peptide ([H] SPSYVYHQF [OH]; Sigma-Genosys) and labeled with 5(6)-carboxyfluorescein diacetate N-succinimidyl ester (CFSE, Sigma-Aldrich). Antigen-pulsed and non-pulsed cells were combined (1:1) and injected into treated mice. The percentage cell killing was determined via flow cytometry 24 h later.

### Results

In the CT26 tumor model, the combination of anti-PD-1 mAb and sunitinib resulted in enhanced *in vivo* cytotoxicity to an antigen-specific immune response (Figure 8). This enhanced cytotoxicity showed an increased immune response component. It also correlated with a reduction in tumor volume (Figure 9).

### EXAMPLE 4

### Phase 1 Clinical Trial to Treat Metastatic RCC with Nivolumab in Combination with Sunitinib, Pazopanib, or Ipilimumab

Collectively, the data disclosed in Examples 1-3 support the investigation of a combination of an anti-PD-1 Ab, such as nivolumab or pembrolizumab, with an anti-angiogenic TKI such as sunitinib, pazopanib, sorafenib, axitinib or tivozanib for treating subjects with RCC. These data suggest that the immunomodulatory properties of sunitinib may lead to changes in the tumor microenvironment that support an effective antitumor immune response, which could be expanded and enhanced by an anti-PD-1 Ab. Although combination therapy with the anti-PD-1 mAb and sorafenib did not significantly enhance the anti-tumor activity of sorafenib in the murine RCC model and did not result in synergistic efficacy (Figure 1), the combination was well tolerated and could be effective in human patients. The safety and efficacy of nivolumab combined with sunitinib, pazopanib, or ipilimumab are currently being assessed in an ongoing Phase 1 study (NCT01472081) in subjects with mRCC. Pazopanib is an orally available multikinase inhibitor (VEGFR-1, VEGFR-2, VEGFR-3, PDGFR-α and -β, and c-KIT), and the combination of nivolumab and ipilimumab has shown significantly enhanced efficacy in the treatment of melanoma compared to monotherapy with nivolumab (Wolchok et al., 2013; WO 2013/173223).

The primary objectives of this Phase 1 study are to assess the overall safety and tolerability of nivolumab plus sunitinib, pazopanib or ipilimumab to determine the MTD and recommended Phase 2 dose of each combination in RCC patients. Secondary objectives are to assess the preliminary antitumor activity of the combinations, and an exploratory objective is to determine whether response correlates with baseline PD-L1 expression.

### Methods

### Study Design

A Phase 1 open-label study of nivolumab plus sunitinib, pazopanib or ipilimumab in subjects with mRCC is ongoing. The trial includes 5 parallel treatment arms: nivolumab plus sunitinib (Arm S), nivolumab plus pazopanib (Arm P), nivolumab 3 mg/kg plus ipilimumab 1 mg/kg (Arm 1-1), nivolumab 1 mg/kg plus ipilimumab 3 mg/kg (Arm 1-3), and nivolumab 3 mg/kg plus ipilimumab 3 mg/kg (Arm IN-3). Treatment for Arm S and Arm P is being conducted in two parts dose escalation (previously treated subjects) followed by dose expansion (treatment-naïve subjects). Treatment for the I-1 and 1-3 arms is a single initial cohort in each arm consisting of both previously treated as well as treatment-naive subjects. Expansion cohorts of arms I-1 and 1-3, as well as arm IN-3 consists of minimally treated (including treatment-naive) subjects. The study design for Arm S and Arm P is summarized in Figure 10A, and the study design for Arms I-1, I-3 and IN-3 is summarized in Figure 10B.

### Study Population

The study population consists of subjects ≥ 18 years old with histologically confirmed metastatic renal cell carcinoma with a clear-cell component who have at least one measurable lesion as defined by Response Evaluation Criteria for Solid Tumors (RECIST) 1.1. Non-clear cell RCC subjects, with measurable lesion as defined by RECIST 1.1, are allowed in the dose escalation cohorts of Arm S and Arm P only. Favorable or intermediate-risk MSKCC (Memorial Sloan Kettering Cancer Center) prognostic score and Kamofsky performance status (KPS) ≥ 80% are also required for Arms S, P, and 1-1 and 1-3 original cohorts, (for Arms I-1 and 1-3 expansion and IN-3 any MSKCC prognostic score is acceptable).

### Arm S and Arm P

Subjects must have received at least one prior systemic therapy regimen in the advanced/metastatic setting to be eligible for the dose-escalation part of the study, while subjects must be treatment-naive to be eligible for the dose-expansion part of the study. Subjects participating in the dose-escalation phase who received prior pazopanib are assigned to receive nivolumab plus sunitinib, while subjects who received prior sunitinib are assigned to receive nivolumab plus pazopanib. Subjects who have received both prior pazopanib and sunitinib are not eligible. Also excluded from the study are subjects chronically treated with systemic steroids or any other immunosuppressive agents, or those previously treated with anti-PD-1, anti-PD-Ll, or anti-PD-L2, anti-CD137, anti-CTLA-4, or similar agent.

Subjects who have not received prior sunitinib or pazopanib may enroll on either dose-escalation arm and are assigned to these arms in an alternating fashion when both arms are open. Approximately 36 subjects (18 subjects per arm) are expected to be treated in the dose-escalation part of the study. At completion of the escalation cohorts, MTD is assessed based on the modified toxicity probability interval (mTPI) design. If, in either Arm S or Arm P, the MTD of nivolumab is determined to be < 5 mg/kg, the arm will not be further expanded to collect safety data in treatment-naive subjects. If the MTD of nivolumab in combination with sunitinib or pazopanib is determined to be 5 mg/kg or higher, separate expansion cohorts in the respective arm(s) will be opened for enrollment at the 5 mg/kg dose. In the expansion cohort(s) of Arm S and Arm P, only treatment-naïve subjects are enrolled so that approximately 20 treatment-naive subjects are treated per arm. Each treatment cycle is 6 weeks in duration, with nivolumab dosed on Days 1 and 22 and anti-angiogenic therapy dosed according to the approved product label for sunitinib (50 mg daily; 4 weeks on, 2 weeks off) and pazopanib (800 mg daily).

### Dose escalation for Arm S and Arm P

Dose levels are defined in Tables 2 and 3. Intra-patient dose escalation of nivolumab is not permitted.

**Table 2. Doselevels for dose-escalation of nivolumab plus sunitinib**

| Dose Level | Nivolumab (mg/kg) | Sunitinib (mg) |
|---|---|---|
| -1^{a} | 0.3 | 50 |
| 1 | 2.0 | 50 |
| 2 | 5.0 | 50 |

| | | |
|---|---|---|
| ^{a}Dose Level -1 will only be enrolled if de-escalation is required at dose level 1 | | |

**Table 3. Dose levels for dose-escalation of nivolumab plus pazopanib**

| Dose Level | Nivolumab (mg/kg) | Pazopanib (mg) |
|---|---|---|
| -1^{a} | 0.3 | 800 |
| 1 | 2.0 | 800 |
| 2 | 5.0 | 800 |

| | | |
|---|---|---|
| ^{a}Dose Level -1 will only be enrolled if de-escalation is required at dose level 1 | | |

The starting dose of nivolumab for each dose-escalation arm is 2 mg/kg. Initially, 7 eligible subjects per arm were treated at Dose Level 1. A decision as to whether additional subjects will be treated at the same dose level or escalate to a higher dose level (5 mg/kg) will be guided by the number of dose-limiting toxicities (DLTs) observed during the DLT observation period. Subjects who do not complete the DLT period for reasons other than dose-limiting toxicities will be replaced. Dose Level -1 (0.3 mg/kg) may be considered if the safety and tolerability profile for 2 mg/kg is not acceptable.

As a Phase 1 dose-escalation trial for Arm S and Arm P, the sample size at each dose in these arms depends on observed toxicity and posterior inference. A total of 18 subjects are expected to be treated during the dose-escalation phase in each arm. However, there may be more than 18 subjects treated per arm due to replacement, or less than 18 subjects treated per arm due to toxicity at the lowest dose level.

### Dose expansion for Arm S and Arm P

Once the safety profile and tolerability of all doses tested have been characterized and the MTD of combined administration of nivolumab plus sunitinib and nivolumab plus pazopanib has been defined for a given treatment arm, cohort expansion is initiated at 5mg/kg for that arm only if the nivolumab dose at the MTD is 5 mg/kg or higher. If, in any arm, the MTD of nivolumab is determined to be < 5 mg/kg, the arm will not be further expanded. An additional 20 subjects per treatment arm will be treated at the 5 mg/kg dose level for nivolumab in combination with sunitinib or pazopanib to gain additional safety information.

### For Arm I-1 and I-3 (first cohort)

Both prior-treated and treatment-naive subjects are eligible to be enrolled, with approximately 20 subjects treated in each arm, but subjects previously treated with anti-PD-1, anti-PD-Ll, or anti-PD-L2, anti-CD137, anti-CTLA-4, or other similar agent were excluded. Doses for the I-1, I-3 and IN-3 arms are detailed below in Table 4. Intra-patient dose escalation or de-escalation is not permitted for either drug.

**Table 4. Dose levels for combinations of nivolumab plus ipilimumab**

| Arm | Nivolumab (mg/kg) | Ipilimumab (mg/kg) |
|---|---|---|
| 1-1 | 3.0 | 1.0 |
| I-3 | 1.0 | 3.0 |
| IN-3 | 3.0 | 3.0 |

Each treatment cycle is 6 weeks in duration. Nivolumab and ipilimumab are dosed every 3 weeks for four doses, then nivolumab is dosed every 2 weeks starting 3 weeks after the 4th dose of induction therapy or after Day 113 if the 4th dose of induction therapy has not been administered due to treatment delays.

Approximately 20 subjects per arm will be enrolled and treated in the initial cohort of subjects in I-1 and 1-3. These arms will be expanded to a total of 45 subjects treated each arm (with additional 25 subjects treated in expansion cohorts each arm) to better evaluate the safety and efficacy.

### For Arm I-1, 1-3 (expansion cohort) and Arm IN-3

Minimally treated (including treatment-naive) subjects are eligible to be enrolled, with approximately 25 subjects treated in each Arm. Each treatment cycle is 6 weeks in duration. Nivolumab and ipilimumab are dosed every 3 weeks for four doses, then nivolumab is dosed every 2 weeks starting 3 weeks after the 4th dose of induction therapy or after Day 113 if the 4th dose of induction therapy has not been administered due to treatment delays.

### Assignment of prior-treated subjects to treatment arms

Subjects who have received prior treatment in the advanced or metastatic setting are assigned to the dose-escalation cohorts of Arm S and P as described above. Subjects who have received at least one prior systemic therapy, provided they do not qualify for escalation-phase Arm S and/or P when those arms are open, are assigned in an alternating fashion to the two ipilimumab combination arms (1-1 and 1-3) when both of these arms are open. If only one arm is open, these subjects are assigned to the open arm.

### Assignment of treatment-naïve subjects to treatment arms

If all four arms (expansion Arm S and P; I-1 and 1-3) are open for enrollment, treatment-naive subjects are randomly assigned to one of the open arms in a 1:1:1:1 ratio until enrollment is complete.

### Assignment of minimally treated subjects to treatment arms

For 1-1 and 1-3 expansion and IN-3, minimally treated (including treatment-naive) subjects are randomly assigned to one of the open arms in a 1:1:1 ratio until enrollment is complete. Adverse events are graded according to NCI CTCAE v4.0. Disease assessments take place every 6 weeks (± 1 week) from the first dose of study drug for the first four assessments, and then every 12 weeks (± 1 week) until disease progression. Response evaluation is performed according to RECIST 1.1. For the dose-escalation phase of Arm S and Arm P, subjects are treated until unacceptable toxicity, disease progression, or withdrawal of informed consent.

### Study Assessments

### Safety outcome measures

Safety/tolerability outcome measures and determination of MTD for the combinations are the primary endpoints for this study. Key safety assessments include (1) incidence of adverse events (AEs) occurring up to 100 days after the last dose of study drug (or longer for drug-related AEs that have not resolved, stabilized, returned to baseline or been deemed irreversible by 100 days after the last dose), (2) incidence of serious adverse events (SAEs) occurring up to 100 days after the last dose of study drug (or longer for drug-related SAEs that have not resolved, stabilized, returned to baseline or been deemed irreversible by 100 days after the last dose), and (3) frequency of laboratory test by worst toxicity grade including hematology, serum chemistry, and urinalysis tests (assessed at screening, baseline, Days 1, 8, 22, and 29 of cycles 1-4, Days 1 and 22 of cycle 5 for Arm S and Arm P; assessed at screening, baseline, Days 1, 8, 22, and 29 of cycles 1-2, Days 1, 15 and 29 of cycle 3+ for Arm 1-1 and Arm 1-3; assessed at end of treatment and follow-up visit 100 days after last dose for all arms).

### Efficacy outcome measures

Antitumor activity is the secondary endpoint for this study and is measured by objective response rate (ORR) and duration of response (DOR) based on RECIST 1.1. Tumor response is based on tumor assessment at screening, every 6 weeks (± 1 week) from the first dose of study drug for the first 4 assessments and then every 12 weeks (± 1 week) thereafter until disease progression.

### EXAMPLE 5

### Antitumor Activity Exhibited in S and P Arms

Seven subjects were treated on each of arms S N2 and S N5, receiving nivolumab (2 or 5 mg/kg, respectively) in combination with S (50 mg, 4 weeks on, 2 weeks off) until progression or unacceptable toxicity. No DLTs were observed and MTD was not reached. Thus, based on tolerability, the S N5 arm was expanded by 19 additional treatment-naive patients (total n=33 for S arms). Arm P enrolled 20 subjects at N2, receiving 2 mg/kg nivolumab in combination with P (800 mg daily). Four 4 DLTs (elevated ALT/AST [n=3], fatigue [n=1]) were observed, leading to closure of this arm. The dose escalation performed is illustrated in Figure 11.

Seven subjects in the S N2 arm, 26 in the S N5 arm, and 20 in the P N2 arm were evaluable for response by the March 2014 database lock. Clinical activities (ORs: confirmed complete [CR] or partial [PR] responses) were observed in all arms as shown in Table 5. The number of evaluable subjects treated with sunitinib and 2 mg/kg nivolumab is small (7 patients), but 1 (14%) of these showed a complete response, 5 (71%) showed partial responses, and 1 (14%) had stable disease. Overall, of the 33 evaluable subjects treated with sunitinib and both concentrations of nivolumab (2 mg/kg and 5 mg/kg), 18 (55%) showed a confirmed objective response (OR). Of the 20 evaluable subjects treated with pazopanib and 2 mg/kg nivolumab, 9 (45%) showed a confirmed OR. Responses occurred by first assessment (6 weeks) in 41% (arm S) and 56% (arm P) of responding subjects. ORR and corresponding 2-sided exact 95% CIs were calculated by the method of Clopper and Pearson.

**Table 5. Tumor response in RCC patients treated with nivolumab and sunitinib or pazopanib**

| | SUN + NIV2 (N = 7) | SUN + NIV5 (N=26) | SUN + NIV (N = 33) | PAZ + NIV2 (N=20) |
|---|---|---|---|---|
| BOR | | | | |
| CR, n (%) | 1(14) | 0 | 1(3) | 0 |
| PR, n (%) | 5(71) | 12(46) | 17(51) | 9(45) |
| Unconfirmed CR, n | 0 | 0 | 0 | 0 |
| Unconfirmed PR, n (%) | 0 | 0 | 0 | 0 |
| SD, n (%) | 1(14) | 9(35) | 10(30) | 7(35) |
| PD, n (%) | 0 | 1(4) | 1(3) | 4(20) |
| UD, n (%) | 0 | 4(15) | 4(12) | 0 |
| Confirmed ORR (A), n (%) | 6(86) | 12(46) | 18(55) | 9(45) |
| 95% CI | (42.1-99.6) | (26.6-66.6) | (36.4-71.9 | (23.1-68.5) |
| Overall ORR (B), n (%) | 6(86) | 12(46) | 18(55) | 9(45) |
| 95% CI | (42.1-99.6) | (26.6-66.6) | (36.4-71.9) | (23.1-68.5) |

| | SUN + NIV2 (N = 7) | SUN + NIV5 (N = 26) | SUN + NIV (N = 33) | PAZ + NIV2 (N=20) |
|---|---|---|---|---|
| Median DOR, weeks | | | 66.3 | 30.1 |
| (95% CI) | | | (18.1-118+)^{a} | (12.1-117.11+)^{b} |

| | | | | |
|---|---|---|---|---|
| BOR: Best Overall Response; CR: Complete Response; PR: Partial Response; SD: Stable Disease; PD: Progressive Disease; UD: Unable to Determine; CI: Confidence Interval; (A): CR only; (B): CR + Unconfirmed Response; Unconfirmed Response: Ongoing subjects with tumor response indicated at last tumor assessment; DOR: Duration of Response. | | | | |

Duration of response was 18.1-118++ weeks in arm S and 12.1 to 117.1++ weeks in arm P. Stable disease rate was 30% (n=10) in arm S and 35% (n=7) in arm P. Median duration of response and corresponding 2-sided 95% CIs were determined using the Kaplan-Meier method.

The effects on tumor burden for the evaluable subjects in the S and P arms are illustrated by spider plots (Figures 12A-C) and a waterfall plot (Figure 12D).

The confirmed ORRs shown in Table 5, ranging from 45% for the PAZ + NIV2 arm to 86% for the SUN + NIV2 arm are significantly higher than the ORRs seen with nivolumab or sunitinib or pazopanib monotherapy. For example, WO 2013/173223 discloses an ORR of about 28-31% with nivolumab (1 or 10 mg/kg) alone for treatment of RCC in a Phase 1 study. In more recent analyses, Motzer *et al.* (2014) report ORRs of 20, 22 and 20% in a Phase 2 trial for treatment of metastatic RCC with nivolumab administered to the patients at 0.3, 2 and 10.mg/kg, respectively. Choueiri *et al.* (2014) report that nivolumab administered at 0.3, 2 and 10 mg/kg produced ORRs of 9, 23 and 22%, respectively, in metastatic RCC patients pretreated with 1-3 prior anti-angiogenic therapies, whereas 10 mg/kg nivolumab produced an ORR of 13% in treatment-naive patients. For the TKIs, a historical ORR of about 27.5% has been observed with sunitinib alone (*see, e.g.,* Motzer *et al.,* 2007; 2013) and about 30% with pazopanib alone (*see, e.g.,* Sternberg *et al.,* 2010; Motzer *et al.,* 2013).

At first assessment (6 weeks), 7 of 17 subjects (41.2%) in the S arm were responders, whereas 5 of 9 subjects (55.6%) in the P arm were responders. Ongoing responders comprise 10 out of 17 subjects (58.8%) in the S arm and 3 of 9 subjects (33.3%) in the P arm.

### Progression-free survival in S and P Arms

A plot of the proportion of progression-free survival (PFS) versus time is shown in Figure 13. PFS rates at 24 weeks were 79% for arm S and 55% for arm P. The median PFS for the S arm (S + N; n=33) was 55.3 weeks (95% CI 47.9-72.4 weeks) and for the P arm (P + N; n=20) was 31.4 weeks (95% CI 12.1-48.1 weeks).

### Objective response rate by baseline PD-L1 expression

Tumor tissue collection was retrospective (33 samples were available for the S arm and 20 samples for the P arm). Surface PD-L1 expression was measured by a Dako immunohistochemical assay using rabbit anti-human PD-L1 mAb, 28-8 (WO 2013/173223). The cut-off for a positive PD-L1 signal was either 1% or 5% of tumor membrane staining. The ORR observed in patients in the two arms, classified according to PD-L1 expression status, is shown in Table 6.

**Table 6. Objective response rate (ORR) in RCC patients treated with the combination of nivolumab and sunitinib or pazopanib, and PD-L1 tumor expression status**

| Arm | PD-L1 cut-off | PD-L1 status | ORR^{a}, n/N (%) |
|---|---|---|---|
| S | 1% tumor membrane staining | + | 6/15 (40.0) |
| | | - | 10/14 (71.4) |
| | 5% tumor membrane staining | + | 1/5 (20.0) |
| | | - | 15/24 (62.5) |
| P | 1% tumor membrane staining | + | 3/7 (42.9) |
| | | - | 5/10 (50.0) |
| | 5% tumor membrane staining | + | 1/2 (50.0) |
| | | - | 7/15 (46.7) |

| | | | |
|---|---|---|---|
| ^{a}ORR of PD-L1 evaluable patients; ORR includes complete or partial responders determined by RECIST. | | | |

These data show that the response of RCC patients to the combination of nivolumab and sunitinib or pazopanib does not correlate with tumor expression of PD-L1.

### Adverse events in S and P Arms

Grade 3-4 related adverse events (AEs) were observed in 27/33 patients (82%) in arm S and 14/20 patients (70%) in arm P. Most toxicities were consistent with the known profile of TKIs. The most common related grade 3-4 AEs included elevated ALT and hypertension (18% each), hyponatremia and decreased lymphocyte count (15% each) in arm S, and elevated ALT and AST and diarrhea (20% each) and fatigue (15%) in arm P. Grade 3 pneumonitis occurred in 1 patient (arm S, N5). Grade 3-4-related AEs led to therapy discontinuation in 11/33 patients (33%; 3 N2, 8 N5) in arm S and 4/20 patients (20%) in arm P. No grade 5 treatment-related AEs were observed. Hepatotoxicities were manageable using treatment algorithms.

Overall, the clinical data indicate that the combination of nivolumab and sunitinib exhibits encouraging antitumor activity and a manageable safety profile in patients with mRCC. Arm P was closed due to DLTs.

### EXAMPLE 6

### Antitumor Activity Exhibited in Ipilimumab Arms

Patients with mRCC (favorable or intermediate MSKCC score; Karnofsky performance status ≥ 80%; untreated or any number of prior therapies except prior immuno-oncology treatments) were randomized to different treatment arms. Forty-seven subjects treated with 1 mg/kg ipilimumab and 3 mg/kg nivolumab (1-1 arm), 47 subjects treated with 3 mg/kg ipilimumab and 1 mg/kg nivolumab (1-3 arm), and six subjects treated with 3 mg/kg ipilimumab and 3 mg/kg nivolumab (IN-3) were evaluable for response. Most patients (n=54; 54%) had prior systemic therapy (1-1: 22; 1-3: 26;IN-3:3). ORs were observed in the 1-1 and 1-3 arms as shown in Table 7. ORR and corresponding 2-sided exact 95% CIs were calculated by the method of Clopper and Pearson. Overall, 16 (34%) patients in the 1-1 arm showed a confirmed OR whereas 17 (36%) of patients in the I-3 arm showed a confirmed OR.

These ORRs are significantly higher than the ORR seen with nivolumab alone (WO 2013/173223 Motzer et al., 2014; Choueiri *et al.,* 2014; *see* Example 5) or ipilimumab alone. Treatment of metastatic RCC patients with ipilimumab monotherapy in a Phase 2 trial resulted in an ORR of 5% in cohort A (3 mg/kg loading dose followed by 1 mg/kg every 3 wk) and 12.5% in cohort B (3 mg/kg every 3 wk for all doses) although there were disparities in the characteristics of these 2 groups, preventing direct comparisons of efficacy (Yang *et al.,* 2007).

The median duration of response (DOR) was 53.9 weeks in the 1-3 arm and was not reached in the 1-1 arm. Stable disease (SD) as best overall response was seen in 19 (40%) (I-1) and 18 (38%) (1-3) patients.

The effects on tumor burden for the evaluable subjects are illustrated by spider plots (Figures 14A-C) and a waterfall plot (Figure 14D). A comparison of waterfall plots for subjects treated in the S, P and I arms is shown in Figure 15.

**Table 7. Tumor response in RCC patients treated with nivolumab and sunitinib or pazopanib**

| | IPI1 + NIV3 (N = 47) | IPI3 + NIV1 (N = 47) | IPI3 + NIV3 (N = 6) |
|---|---|---|---|
| BOR, n (%) | | | |
| CR | 1(2) | 1(2) | 0 |
| PR | 15(32) | 16(34) | 0 |
| Unconfirmed CR | 0 | 0 | 0 |
| Unconfirmed PR | 2(4) | 3(6) | 0 |
| SD | 19(40) | 18(38) | 5(83) |
| PD | 9(19) | 7(15) | 1(16.7) |
| UD | 1(2) | 2(4) | 0 |
| Confirmed ORR (A), n (%) | 16(34) | 17(36) | 0 |
| 95% CI | (20.949.3) | (22.7-51.5) | |
| Overall ORR (B), n (%) | 18(38) | 20(43) | 0 |
| 95% CI | (24.5-53.6) | (28.3-57.8) | |
| Median PFS, weeks (95% CI) | 30.3 (4.7+, 72.6+) | 36.0 (4.1 , 77.9+) | NR (7.0,28.1+) |
| 24-week PFS, % (95% CI) | NC | NC | NC |
| Median DOR, weeks | NR | 53.9 | NC |
| (Range)^{a} weeks | (4.1+ - 67.1+) | (6.1+-66.0+) | |
| Ongoing responses, % (n/N) | 94 (15/17) | 71 (12/17) | |

| | | | |
|---|---|---|---|
| BOR: Best Overall Response; CR: Complete Response; PR: Partial Response; SD: Stable Disease; PD: Progressive Disease; UD: Unable to Determine; CI: Confidence Interval; (A): CR only; (B): CR + Unconfirmed Response; Unconfirmed Response: Ongoing subjects with tumor response indicated at last tumor assessment; PFS: Progression-Free Survival, DOR: Duration of Response; NR: Not Reached; ^{a}Due to the high percentage of ongoing responses, median DOR may be misleading. Duration of response is defined as the time between date of first response and date of disease progression or death (whichever occurs first). | | | |

### Progression-free survival in I-1 and I-3 Arms

A plot of the proportion of PFS versus time is shown in Figure 16. The median PFS was 30.3 weeks in the 1-1 arm and 36.0 weeks in the I-3 arm. PFS rate was estimated by Kaplan-Meier methodology with the corresponding 95% CIs derived based on the Greenwood formula.

### Objective response rate by baseline PD-L1 expression

Tumor tissue samples were retrospectively collected, and surface PD-L1 expression was measured by the Dako immunohistochemistry assay using mAb, 28-8 (WO 2013/173223). The cut-off for a positive PD-L1 signal was either 1% or 5% of tumor membrane staining. The ORR observed in different patients, classified according to PD-L1 expression status, is shown in Table 8.

**Table 8. Objective response rate (ORR) in RCC patients treated with the combination of nivolumab and ipilimumab, and PD-L1 tumor expression status**

| PD-L1 cut-off | PD-L1 status | ORR^{a}, n/N (%) |
|---|---|---|
| 1% tumor membrane staining | + | 11/33 (33.3) |
| | - | 18/55 (32.7) |
| 5% tumor membrane staining | + | 3/14 (21.4) |
| | - | 26/74 (35.1) |

| | | |
|---|---|---|
| ^{a}ORR of PD-L1 evaluable patients; ORR includes complete or partial responders determined by RECIST. | | |

These data show that response of RCC patients to the combination of nivolumab and ipilimumab does not correlate with tumor expression of PD-L1.

### Adverse events in I-1 and I-3 Arms

Grade 3-4 related adverse events (AEs) were observed in 16/47 (34%), 30/47 (64%) and 5/6 (83%) in the I1, I3, and IN-3 arms respectively. Five (11%) and 10 (21%) discontinued treatment due to related AE's in the I1 and I3 arms respectively. No patients discontinued due to adverse events in the IN-3 arm, however all six patients required steroids for the treatment of immune-mediated adverse events and not additional patients were enrolled in the IN3 arm.. The most common Grade 3-4 related adverse events included elevated lipase (20%, n=20), elevated ALT (11%, n=11), diarrhea (9%, n=9), and elevated AST (6%, n=6). No grade 3-4 pneumonitis was seen.

Overall, nivolumab and ipilimumab showed acceptable safety, grade 3-4 events being manageable within established treatment guidelines, and encouraging evidence of significant antitumor activity in mRCC, with most responses ongoing. The ORR suggests greater activity than reported previously with nivolumab or ipilimumab monotherapy in RCC (Topalian *et al.,* 2012a; Motzer *et al.,* 2014; Choueiri *et al.,* 2014; Yang *et al.,* 2007). The median duration of response had not been reached in the 1-1 arm. A portion of patients continued to respond off therapy, suggesting an immune memory component to this regimen. The initial and expansion cohorts, along with the IN-3 arm are continuing to be assessed. The encouraging antitumor activity reported with the nivolumab + ipilimumab combinations is the basis for a planned phase III combination trial in first-linc mRCC.

### REFERENCES

Amin et al. (2014) Nivolumab (anti-PD-1; BMS-936558, ONO-4538) in combination with sunitinib or pazopanib in patients (pts) with metastatic renal cell carcinoma (mRCC). J Clin Oncol 32:5s, (suppl; Abstract 5010, Clinical Science Symposium).
Blank et al. (2005) Cancer Immunol. Immunother. 54:307-314.
Brahmer et al. (2010) J Clin Oncol 28:3167-75.
Brahmer et al. (2012) N Engl J Med 366:2455-65.
Carter et al. (2002) Eur J Immunol 32:634-43.
Choueiri et al. (2014) Immunomodulatory activity of nivolumab in previously treated and untreated metastatic renal cell carcinoma (mRCC): Biomarker-based results from a randomized clinical trial. J Clin Oncol 32:5s, (suppl; Abstract 5012, Clinical Science Symposium).
Dong et al. (2002) Nat. Med. 8:787-9.
Dong et al. (2003) J. Mol. Med. 81:281-7.
Drake et al. (2013) BJU Int 112(Supplement 3):1-17.
Elhilali et al. (2000) BJU Int 86:613-8.
Escudier et al. (2007) Clin Cancer Res 13(6):1801-1809.
Escudier et al. (2010) J Clin Oncol 28(13):2144-50.
Eskens et al. (2011) Clin Cancer Res 17(22):7156-63.
Flies et al. (2011) Yale J Biol Med 84:409-21.
Freeman et al. (2000) J Exp Med 192:1027-34.
Fyfe et al. (1995) J Clin Oncol 13(3):688-96.
Gollob et al. (2007) J Clin Oncol 25(22): 3288-95.
Grunwald et al. (2011) Acta Oncol 50(1):121-126.
Gupta et al. (2008) Cancer Treat Rev 34(3):193-205.
Hamid and Carvajal (2013) Expert Opin Biol Ther 13(6):847-61.
Hamid et al. (2013) N Engl J Med 369:134-144.
Hammers et al. (2014) Phase I study of nivolumab in combination with ipilimumab in metastatic renal cell carcinoma (mRCC). J Clin Oncol 32:5s, (suppl; Abstract 4504, Oral Abstract Session).
Heng et al. (2009) J Clin Oncol. 27:5794-9.
Herbst et al. (2013) J Clin Oncol 31(suppl; Abstract 3000).
Hipp et al. (2008) Blood 111:5610-20.
Hodi et al. (2010) N Engl J Med 363:711-23.
Houben et al. (2009) Mol Cancer Ther 28:433-40.
Hudes et al. (2007) N Engl J Med 356(22):2271-81.
Inman et al. (2013) Eur Urol 63:881-9.
Konishi et al. (2004) Clin. Cancer Res. 10:5094-100.
Johnson et al. (2013) Cancer Immunol Res 1:373-77.
Khleif (2013) In: Proceedings from the European Cancer Congress 2013; September 27-October 1, 2013; Amsterdam, The Netherlands. Abstract 802.
Ko et al. (2009) Clin Cancer Res 15:2148-57.
Ko et al. (2010) Cancer Res 70:3526-36.
Konishi et al. (2004) Clin. Cancer Res. 10:5094-100.
Latchman et al. (2001) Nat Immunol 2:261-8.
McDermott and Atkins (2013) Cancer Med 2(5):662-73.
Molhoek et al. (2009) Cancer Immunol Immunother 58:867-76.
Motzer et al. (2007) N Engl J Med 356:115-24.
Motzer et al. (2008) Lancet 372:449-56.
Motzer et al. (2009) Clin Genitourin Cancer 7(1):28-33.
Motzer et al. (2013) N Engl J Med 369:722-31.
Motzer et al. (2014) Nivolumab for metastatic renal cell carcinoma (mRCC): Results of a randomized, dose-ranging phase II trial. J Clin Oncol 32:5s, (suppl; Abstract 5009, Clinical Science Symposium).
Mulders (2009) BJU Int 104:1585-89.
NCCN GUIDELINES® (2014), available at: http://www.nccn.org/professionals/physician_gls/f_guidelines.asp#site, last accessed May 30, 2014.
Nosov et al. (2012) J Clin Oncol 30(14):1678-85.
Ozao-Choy et al. (2009) Cancer Res 69:2514-22.
Pardoll (2012) Nat Rev Cancer 12:252-64.
PCT Publication No. WO 2012/145493, published October 26, 2012 by Amplimmune, Inc.
PCT Publication No. WO 2013/173223, published November 21, 2013 by Bristol-Myers Squibb Co.
Procopio et al. (2011) Br J Cancer 104(8):1256-1261.
Ribas (2010) Semin Oncol 37(5):450-4.
Rini et al. (2010) J Clin Oncol 28(13): 2137-43.
Rini et al. (2011) Cancer 117:758-67.
Rosenblatt and McDermott (2011) Hematol Oncol Clin North Am 25:793-812.
Ryan et al. (2007) J Clin Oncol 25(22):3296-301.
Siegel et al. (2014) CA Cancer J Clin 64(1):9-29.
Sjoblom et al. (2006) Science 314:268-74.
Sonpavde et al. (2008) Expert Opin Investig Drugs 17(5):741-8.
Sternberg et al. (2010) J Clin Oncol 28(6):1061-8.
Topalian et al. (2012a) N Engl J Med 366:2443-54.
Topalian et al. (2012b) Curr Opin Immunol 24:207-12.
Topalian et al. (2014) J Clin Oncol 32(10):1020-30.
USAN Council Statement (2013) Pembrolizumab: Statement on a nonproprietary name adopted by the USAN Council (ZZ-165), November 27, 2013.
U.S. Patent No. 5,977,318, issued November 2, 1999 to Linsley et al.
U.S. Patent No. 6,051,227, issued April 18, 2000 to Allison et al.
U.S. Patent No. 6,682,736, issued January 27, 2004 to Hanson et al.
U.S. Patent No. 6,808,710, issued October 26, 2004 to Wood et al.
U.S. Patent No. 6,984,720, issued January 10, 2006 to Korman et al.
U.S. Patent No. 7,034,121, issued April 25, 2006 to Carreno et al.
U.S. Patent No. 7,605,238, issued October 20, 2009 to Korman et al.
U.S. Patent No. 7,488,802, issued February 10, 2009 to Collins et al.
U.S. Patent No. 7,943,743, issued May 17, 2011 to Korman et al.
U.S. Patent No. 8,008,449, issued August 30, 2011 to Korman et al.
U.S. Patent No. 8,168,757, issued May 1, 2012 to Finnefrock et al.
U.S. Patent No. 8,217,149, issued July 10, 2012 to Irving et al.
U.S. Patent No. 8,354,509, issued January 15, 2013 to Carven et al.
Yang et al. (2007) J Immunother 30(8):825-30.
Wang et al. (2014) In vitro characterization of the anti-PD-1 antibody nivolumab, BMS-936558, and in vivo toxicology in non-human primates, Cancer Imm Res*,* in press.
Wolchok et al. (2013) N Engl J Med 369(2):122-33.

The following embodiments El to E49 form part of the invention:
1. A method for treating a subject afflicted with a renal cancer comprising administering to the subject a combination of therapeutically effective amounts of:
   (a) an anti-cancer agent which is an antibody or an antigen-binding portion thereof that binds specifically to a Programmed Death-1 (PD-1) receptor and inhibits PD-1 activity; and
   (b) another anti-cancer agent.
2. The method of embodiment 1, wherein the renal cancer is renal cell carcinoma.
3. The method of embodiment 1, wherein the anti-PD-1 antibody or antigen-binding portion thereof cross-competes with nivolumab for binding to human PD-1.
4. The method of embodiment 1, wherein the anti-PD-1 antibody or antigen-binding portion thereof is a chimeric, humanized or human monoclonal antibody or a portion thereof.
5. The method of any one of embodiments 1-4, wherein the anti-PD-1 antibody or antigen-binding portion thereof comprises a heavy chain constant region which is of a human IgG1 or IgG4 isotype.
6. The method of embodiment 1, wherein the anti-PD-1 antibody is nivolumab.
7. The method of embodiment 1, wherein the anti-PD-1 antibody is pembrolizumab.
8. The method of embodiment 1, wherein the other anti-cancer agent is an anti-angiogenic tyrosine kinase inhibitor.
9. The method of embodiment 8, wherein the anti-PD-1 antibody or antigen-binding portion thereof is administered at a dose ranging from about 0.1 to about 10.0 mg/kg body weight once about every 2, 3 or 4 weeks.
10. The method of embodiment 9, wherein the anti-PD-1 antibody or antigen-binding portion thereof is administered at a dose of 2 or 5 mg/kg body weight once every 3 weeks.
11. The method of embodiment 8, wherein the tyrosine kinase inhibitor is sunitinib.
12. The method of embodiment 11, wherein the sunitinib is administered at a dose ranging from 12.5 to 50 mg in a cycle comprising daily administration for 28 days and no administration for 14 days, and repetition of the cycle for as long as clinical benefit is observed or until unmanageable toxicity or disease progression occurs.
13. The method of embodiment 11, wherein the sunitinib is administered at a dose ranging from 12.5 to 50 mg in a cycle comprising daily administration for 28 days and no administration for 14 days, followed by daily administration of sunitinib at about 37.5 mg for as long as clinical benefit is observed or until unmanageable toxicity or disease progression occurs.
14. The method of embodiment 8, wherein the tyrosine kinase inhibitor is pazopanib.
15. The method of embodiment 14, wherein the pazopanib is administered at a dose ranging from 400 to 800 mg administered daily or every other day for as long as clinical benefit is observed or until unmanageable toxicity or disease progression occurs.
16. The method of embodiment 1, wherein the other anti-cancer agent is an antibody or an antigen-binding portion thereof that binds specifically to Cytotoxic T-Lymphocyte Antigen-4 (CTLA-4) and inhibits CTLA-4 activity.
17. The method of embodiment 16, wherein the anti-CTLA-4 antibody or antigen-binding portion thereof cross-competes with ipilimumab for binding to human CTLA-4.
18. The method of embodiment 16, wherein the anti-CTLA-4 antibody or antigen-binding portion thereof is a chimeric, humanized or human monoclonal antibody or a portion thereof.
19. The method of any one of embodiments 16-18, wherein the anti-CTLA-4 antibody or antigen-binding portion thereof comprises a heavy chain constant region which is of a human IgG1 isotype.
20. The method of embodiment 16, wherein the anti-CTLA-4 antibody is ipilimumab.
21. The method of embodiment 16, wherein the anti-CTLA-4 antibody is tremelimumab.
22. The method of embodiment 16, comprising:
   (a) an induction phase, wherein the anti-PD-1 and anti-CTLA-4 antibodies or antigen-binding portions thereof are administered in combination in 2, 4, 6, 8 or 10 doses, each dose ranging from 0.1 to 10.0 mg/kg body weight administered at least once every 2, 3, or 4 weeks; followed by
   (b) a maintenance phase, wherein no anti-CTLA-4 antibody or antigen-binding portion thereof is administered and the anti-PD-1 antibody or antigen-binding portion thereof is repeatedly administered at a dose ranging from 0.1 to 10 mg/kg at least once every 2, 3 or 4 weeks.
23. The method of embodiment 22, wherein:
   (a) the induction phase comprises 4 combination doses administered at 3-week intervals, wherein:
      (i) the anti-PD-1 antibody or antigen-binding portion thereof is administered at 3 mg/kg body weight and the anti-CTLA-4 antibody or antigen-binding portion thereof is administered at 1 mg/kg body weight;
      (ii) the anti-PD-1 antibody or antigen-binding portion thereof is administered at 1 mg/kg body weight and the anti-CTLA-4 antibody or antigen-binding portion thereof is administered at 3 mg/kg body weight; or
      (iii) the anti-PD-1 antibody or antigen-binding portion thereof is administered at 3 mg/kg body weight and the anti-CTLA-4 antibody or antigen-binding portion thereof is administered at 3 mg/kg body weight; and
   (b) the maintenance phase comprises repeated administration of the anti-PD-1 antibody or antigen-binding portion thereof at a dose of 3 mg/kg every 2 weeks for as long as clinical benefit is observed or until unmanageable toxicity or disease progression occurs.
24. The method of any embodiment 22, wherein the anti-PD-1 and anti-CTLA-4 antibodies are formulated for intravenous administration.
25. The method of embodiment 22, wherein the anti-PD-1 antibody or antigen-binding portion thereof and the anti-CTLA-4 antibody or antigen-binding portion thereof are administered sequentially to the subject during the induction phase.
26. The method of embodiment 25, wherein the anti-PD-1 and anti-CTLA-4 antibodies are administered within 30 minutes of each other.
27. The method of embodiment 25, wherein
   (a) the anti-PD-1 antibody or antigen-binding portion thereof is administered before the anti-CTLA-4 antibody or antigen-binding portion thereof; or
   (b) the anti-CTLA-4 antibody or antigen-binding portion thereof is administered before the anti-PD-1 antibody or antigen-binding portion thereof.
28. The method of embodiment 22, wherein the anti-PD-1 antibody or antigen-binding portion thereof and the anti-CTLA-4 antibody or antigen-binding portion thereof are administered concurrently in separate compositions.
29. The method of embodiment 22, wherein the anti-PD-1 antibody or antigen-binding portion thereof and the anti-CTLA-4 antibody or antigen-binding portion thereof are admixed as a single composition for concurrent administration.
30. The method of embodiment 22, wherein the anti-PD-1 antibody or antigen-binding portion thereof is administered at a subtherapeutic dose.
31. The method of embodiment 22, wherein the anti-CTLA-4 antibody or antigen-binding portion thereof is administered at a subtherapeutic dose.
32. The method of embodiment 22, wherein the anti-PD-1 antibody or antigen-binding portion thereof and the anti-CTLA-4 antibody or antigen-binding portion thereof are each administered at a subtherapeutic dose.
33. The method of embodiment 22, wherein administration of the anti-PD-1 antibody in the maintenance phase is continued for as long as clinical benefit is observed or until unmanageable toxicity or disease progression occurs.
34. A kit for treating a subject afflicted with a renal cancer, the kit comprising:
   (a) a dosage ranging from 0.1 to 10 mg/kg body weight of an anti-cancer agent which is an antibody or an antigen-binding portion thereof that specifically binds to the PD-1 receptor and inhibits PD-1 activity;
   (b) a dosage of another anti-cancer agent which is
      (i) an anti-angiogenic tyrosine kinase inhibitor; or
      (ii) a dosage ranging from 0.1 to 10 mg/kg body weight of an antibody or an antigen-binding portion thereof that specifically binds to and inhibits CTLA-4; and
   (c) instructions for using the anti-PD-1 antibody and the other anti-cancer agent in the method of any of embodiments 1, 8 or 16.
35. The method of any one of embodiments 1 to 33, wherein the administration of the anti-PD-1 antibody or antigen-binding portion thereof and the other anti-cancer agent increases T cell infiltration of the renal cancer tissue compared to an untreated subject or a subject treated with a monotherapy of the anti-PD-1 antibody or antigen-binding portion thereof or the other anti-cancer agent.
36. The method of embodiment 35, wherein the increased T cell infiltration is characterized by increased infiltration of CD4⁺/Ki67⁺ T cells, CD8⁺ T cells, CD8⁺/Ki67⁺ T cells, or any combination thereof.
37. The method of embodiment 36, wherein the increased T cell infiltration is characterized by increased infiltration of CD4⁺/Ki67⁺ T cells, CD8⁺/Ki67⁺ T cells, or both.
38. The method of any one of embodiments 35 to 37, wherein the T cell infiltration is increased by at least about 10%, at least about 20%, at least about 30%, at least about 40%, at least about 50%, at least about 60%, at least about 70%, at least about 80%, or at least about 90%.
39. The method of any one of embodiments 1 to 33 and 35 to 38, wherein the administration of the anti-PD-1 antibody or antigen-binding portion thereof and the other anti-cancer agent increases proliferating T cells compared to an untreated subject or a subject treated with a monotherapy of the anti-PD-1 antibody or antigen-binding portion thereof or the other anti-cancer agent.
40. The method of embodiment 39, wherein the proliferating T cells are CD4⁺/Ki67⁺ T cells, CD8⁺ T cells, CD8⁺/Ki67⁺ T cells, or any combination thereof.
41. The method of embodiment 39 or 40, wherein the frequency of proliferating T cells in increased by at least about 10%, at least about 20%, at least about 30%, at least about 40%, at least about 50%, at least about 60%, at least about 70%, at least about 80%, or at least about 90%.
42. The method of any one of embodiments 1 to 33 and 35 to 41, wherein the administration of the anti-PD-1 antibody or antigen-binding portion thereof and the other anti-cancer agent decreases the number of monocytic myeloid-derived suppressor cells compared to an untreated subject or a subject treated with a monotherapy of the anti-PD-1 antibody or antigen-binding portion thereof or the other anti-cancer agent.
43. The method of embodiment 42, wherein the number of monocytic myeloid-derived suppressor cells is decreased by at least about 5%, at least about 10%, at least about 15%, at least about 20%, at least about 25%, at least about 30%, at least about 40%, at least about 50%, at least about 60%, at least about 70%, at least about 80%, or at least about 90%.
44. The method of embodiment 42 or 43, wherein the monocytic myeloid-derived suppressor cells are characterized by CD11b⁺/Ly6C^{hi}/Ly6G⁻ expression or CD11b⁺/Ly6C^{low}/Ly6G⁻ expression.
45. The method of any one of embodiments 1 to 33 and 35 to 44, wherein the administration of the anti-PD-1 antibody or antigen-binding portion thereof and the other anti-cancer agent increases the number of granulocytic myeloid cells compared to an untreated subject or a subject treated with a monotherapy of the anti-PD-1 antibody or antigen-binding portion thereof or the other anti-cancer agent.
46. The method of embodiment 44, wherein the number of granulocytic myeloid cells is increased by at least about 5%, at least about 10%, at least about 20%, at least about 30%, at least about 40%, at least about 50%, at least about 60%, at least about 70%, at least about 80%, or at least about 90%.
47. The method of embodiment 45 or 46, wherein the granulocytic myeloid cells are characterized by CD11b⁺/Ly6C⁻/Ly6G⁺ expression.
48. The method of any one of embodiments 1 to 33 and 35 to 47, wherein the administration of the anti-PD-1 antibody or antigen-binding portion thereof and the other anti-cancer agent decreases T-regulatory cells compared to an untreated subject or a subject treated with a monotherapy of the anti-PD-1 antibody or antigen-binding portion thereof or the other anti-cancer agent.
49. The method of embodiment 48, wherein the T-regulatory cells are characterized by CD45⁺/CD4⁺/CD25⁺/FoxP3⁺ expression.

## Claims

1. A pharmaceutical composition comprising a therapeutically effective amount of an antibody or an antigen-binding portion thereof that binds specifically to a Programmed Death-1 (PD-1) receptor and inhibits PD-1 activity ("an anti-PD-1 antibody or antigen-binding portion thereof') for treating a subject afflicted with a renal cancer in combination with a therapeutically effective amount of an anti-cancer agent;
wherein the anti-cancer agent is selected from:
(i) axitinib, wherein the axitinib is administered at a dose of about 2 mg, about 3 mg, about 7 mg or about 10 mg orally twice daily;
(ii) sunitinib, wherein the sunitinib is administered at a dose ranging from 12.5 mg to 50 mg in a cycle comprising daily administration for 28 days and no administration for 14 days, followed by daily administration of sunitinib at about 37.5 mg for as long as clinical benefit is observed or until unmanageable toxicity or disease progression occurs;
(iii) pazopanib, wherein the pazopanib is administered at a dose of about 200 mg, about 400 mg, about 600 mg, about 700 mg, about 800 mg, about 900 mg, or about 1000 mg once a day;
(iv) sorafenib, wherein the sorafenib is administered at a dose of about 100 mg, about 200 mg, about 300 mg, about 400 mg, about 500 mg, about 600 mg or about 800 mg twice a day;
(v) tivozanib, wherein the tivozanib is administered at a dose of about 0.5 mg, about 0.75 mg, about 1 mg, about 1.25 mg, about 1.5 mg, about 1.75 mg or about 2 mg once a day; and
(vi) any combination of (i)-(v).

2. The pharmaceutical composition for use of claim 1, wherein the renal cancer is renal cell carcinoma.

3. The pharmaceutical composition for use of claim 1, wherein the anti-PD-1 antibody or antigen-binding portion thereof cross-competes with nivolumab for binding to human PD-1.

4. The pharmaceutical composition for use of claim 1, wherein the anti-PD-1 antibody is nivolumab or pembrolizumab.

5. The pharmaceutical composition for use of claim 1, wherein the anti-PD-1 antibody or antigen-binding portion thereof is administered at a dose ranging from about 0.1 mg/kg to about 10.0 mg/kg body weight once about every 2, 3, or 4 weeks.

6. The pharmaceutical composition for use of claim 5, wherein the anti-PD-1 antibody or antigen-binding portion thereof is administered at a dose of 2 mg/kg or 5 mg/kg body weight once every 3 weeks.

7. A kit for treating a subject afflicted with a renal cancer, the kit comprising:
(a) a dosage ranging from 0.1 mg/kg to 10 mg/kg body weight of an anti-cancer agent which is an antibody or an antigen-binding portion thereof that specifically binds to the PD-1 receptor and inhibits PD-1 activity ("an anti-PD-1 antibody or antigen-binding portion thereof');
(b) a dosage of another anticancer agent; and
(c) instructions for using the anti-PD-1 antibody or antigen-binding portion thereof and the other anticancer agent in the method of claim 1;
wherein the dosage of the other anticancer agent is selected from:
(i) about 2 mg, about 3 mg, about 7 mg or about 10 mg axitinib;
(ii) from 12.5 mg to 50 mg sunitinib;
(iii) about 200 mg, about 400 mg, about 600 mg, about 700 mg, about 800 mg, about 900 mg, or about 1000 mg pazopanib;
(iv) about 100 mg, about 200 mg, about 300 mg, about 400 mg, about 500 mg, about 600 mg or about 800 mg sorafenib;
(v) about 0.5 mg, about 0.75 mg, about 1 mg, about 1.25 mg, about 1.5 mg, about 1.75 mg or about 2 mg tivozanib; and
(vi) any combination of (i)-(v).

8. The pharmaceutical composition for use of any one of claims 1 to 6, wherein the administration of the anti-PD-1 antibody or antigen-binding portion thereof and the anti-cancer agent
(i) increases T cell infiltration of the renal cancer tissue compared to an untreated subject or a subject treated with a monotherapy of the anti-PD-1 antibody or antigen-binding portion thereof or the anti-cancer agent;
(ii) increases proliferating T cells compared to an untreated subject or a subject treated with a monotherapy of the anti-PD-1 antibody or antigen-binding portion thereof or the anti-cancer agent;
(iii) decreases the number of monocytic myeloid-derived suppressor cells compared to an untreated subject or a subject treated with a monotherapy of the anti-PD-1 antibody or antigen-binding portion thereof or the anti-cancer agent;
(iv) increases the number of granulocytic myeloid cells compared to an untreated subject or a subject treated with a monotherapy of the anti-PD-1 antibody or antigen-binding portion thereof or the anti-cancer agent; or
(v) decreases T-regulatory cells compared to an untreated subject or a subject treated with a monotherapy of the anti-PD-1 antibody or antigen-binding portion thereof or the anti-cancer agent.

9. The pharmaceutical composition for use of claim 8, wherein
(i) the increased T cell infiltration is **characterized by** increased infiltration of CD4⁺/Ki67⁺ T cells, CD8⁺ T cells, CD8⁺/Ki67⁺ T cells, or any combination thereof;
(ii) the proliferating T cells are CD4⁺/Ki67⁺ T cells, CD8⁺ T cells, CD8⁺ /Ki67⁺ T cells, or any combination thereof;
(iii) the monocytic myeloid-derived suppressor cells are **characterized by** CD11b⁺/Ly6C^{hi}/Ly6G⁻ expression or CD11b⁺/Ly6C^{low}/Ly6G⁻ expression;
(iv) the granulocytic myeloid cells are **characterized by** CD11b⁺/Ly6C⁻/Ly6G⁺ expression; or
(v) the T-regulatory cells are **characterized by** CD45⁺/CD4⁺/CD25⁺/FoxP3⁺ expression.

10. The pharmaceutical composition for use of claim 8 or 9, wherein
(i) the T cell infiltration is increased by at least about 10%, at least about 20%, at least about 30%, at least about 40%, at least about 50%, at least about 60%, at least about 70%, at least about 80%, or at least about 90%;
(ii) the frequency of proliferating T cells in increased by at least about 10%, at least about 20%, at least about 30%, at least about 40%, at least about 50%, at least about 60%, at least' about 70%, at least about 80%, or at least about 90%;
(iii) the number of monocytic myeloid-derived suppressor cells is decreased by at least about 5%, at least about 10%, at least about 15%, at least about 20%, at least about 25%, at least about 30%, at least about 40%, at least about 50%, at least about 60%, at least about 70%, at least about 80%, or at least about 90%; and/or
(iv) the number of granulocytic myeloid cells is increased by at least about 5%, at least about 10%, at least about 20%, at least about 30%, at least about 40%, at least about 50%, at least about 60%, at least about 70%, at least about 80%, or at least about 90%.
